# EUROPÄISCHE PATENTSCHRIFT

(11) **EP 1 638 995 B1**
(45) Veröffentlichungstag und Bekanntmachung des Hinweises auf die Patenterteilung: **26.08.2015**
(21) Anmeldenummer: 04739976.1
(22) Anmeldetag: 17.06.2004
(51) Int. Cl.: C07K 14/02, G01N 33/53, A61K 39/29

(54) **NEUE OBERFLÄCHENPROTEIN-(HBSAG-) VARIANTE DES HEPATITIS B VIRUS**
NOVEL SURFACE PROTEIN (HBSAG) VARIANT OF HEPATITIS B VIRUS
NOUVELLE VARIANTE DE LA PROTEINE DE SURFACE (HBSAG-) DU VIRUS DE L'HEPATITE B

(30) Priorität: 20.06.2003 DE 10328139
(43) Veröffentlichungstag der Anmeldung: 29.03.2006
(73) Patentinhaber: Siemens Healthcare Diagnostics Products GmbH, 35041 Marburg (DE)
(72) Erfinder: KRUPKA, Udo, 35043 Marburg (DE)
(86) Internationale Anmeldenummer: PCT/EP2004/006516
(87) Internationale Veröffentlichungsnummer: WO 2004/113370

(56) Entgegenhaltungen:
- WO-A1-99/51753
- WO-A2-00/24782
- WO-A2-01/83692
- WO-A2-02/055741
- WO-A2-2004/071457
- -A-
- US-A1- 2003 194 414
- DATABASE EMBL Large S protein (fragment) 1. Februar 1997 (1997-02-01), XP002297050 gefunden im EBI Database accession no. Q96841
- DATABASE EMBL Hepatitis B virus s gene 27. Juni 2003 (2003-06-27), XP002297051 gefunden im EBI Database accession no. AB104715
- DATABASE EMBL Hepatitis B virus DNA complete sequence 10. Juli 2000 (2000-07-10), XP002297066 gefunden im EBI Database accession no. AF280817
- DATABASE EMBL Hepatitis B virus partial S gene 6. Januar 2001 (2001-01-06), TIGEN: XP002297056 gefunden im EBI Database accession no. AJ297881
- DATABASE EMBL Hepaptitis B virus strain 926h preS2-S protein 6. Februar 2000 (2000-02-06), XP002297053 gefunden im EBI Database accession no. AF214660 & BORCHANI-CHABCHOUB I ET AL: "Genotyping of Tunisian hepatitis B virus isolates based on the sequencing of preS2 and S regions" MICROBES AND INFECTION, ELSEVIER, PARIS, FR, Bd. 2, Nr. 6, Mai 2000 (2000-05), Seiten 607-612, XP002229227 ISSN: 1286-4579
- CARMAN W F ET AL: "FULMINANT REACTIVATION OF HEPATITIS B DUE TO ENVELOPE PROTEIN MUTANT THAT ESCAPED DETECTION BY MONOCLONAL HBSAG ELISA" LANCET THE, LANCET LIMITED. LONDON, GB, Bd. 345, Nr. 8962, 3. Juni 1995 (1995-06-03), Seiten 1406-1407, XP002041203 ISSN: 0140-6736
- WEINBERGER KLAUS M ET AL: "High genetic variability of the group-specific a-determinant of hepatitis B virus surface antigen (HBsAg) and the corresponding fragment of the viral polymerase in chronic virus carriers lacking detectable HBsAg in serum" JOURNAL OF GENERAL VIROLOGY, Bd. 81, Nr. 5, Mai 2000 (2000-05), Seiten 1165-1174, XP002297178 ISSN: 0022-1317
- CHIOU H-L ET AL: "Altered antigenicity of 'a' determinant variants of hepatitis B virus" JOURNAL OF GENERAL VIROLOGY, SOCIETY FOR GENERAL MICROBIOLOGY, READING, GB, Bd. 78, 1997, Seiten 2639-2645, XP002174817 ISSN: 0022-1317
- DATABASE EMBL [Online] AAM27432, 13 Dezember 2002 'S antigen, partial (Hepatitis B virus)' Database accession no. AAM27432

### SEQUENCE LISTING

<110> Dade Behring Marburg GmbH
<120> Neue Oberflaechenprotein-(HBsAg-) Variante des Hepatitis B Virus
<130> MA 1251
<150> DE 10328139.8
   <151> 2003-06-20
<160> 34
<170> PatentIn version 3.2
<210> 1
   <211> 462
   <212> DNA
   <213> Hepatitis B virus
<400> 1
   gggggaacta ccgtgtgtct tggccaaaat tcgcagtccc caacctccaa tcactcacca     60
   acctcctgtc ctccaacttg tcctggttat cgctggatgt gtctgcggcg ttttatcatc    120
   ttcctcttca tcctgctgct atgcctcatc ttcttgttgg ctcttctgga ctatcaaggt    180
   atattgcccg tttgtcctct aattccagga tctgcaatca acaacagggg acaatgcaaa    240
   acctgcacga ctactgctca cggaacctct atgtatccct actgttgctg taccaaacct    300
   tcggacggaa attgcacctg tattcccatc ccatcatcct gggctttcgg aaaattccta    360
   tgggagtggg cctcagcccg tttctcctgg ctcagtttac tagttccctt tgttcagtgg    420
   ttcgtagggc tttcccccac tgtttggctt tcagttatat gg                       462
<210> 2
   <211> 84
   <212> DNA
   <213> Hepatitis B virus
<400> 2
   ttcttgttgg ctcttctgga ctatcaaggt atattgcccg tttgtcctct aattccagga     60
   tctgcaatca acaacagggg acaa                                             84
<210> 3
   <211> 144
   <212> DNA
   <213> Hepatitis B virus
<400> 3
   ttcttgttgg ctcttctgga ctatcaaggt atattgcccg tttgtcctct aattccagga     60
   tctgcaatca acaacagggg acaatgcaaa acctgcacga ctactgctca cggaacctct    120
   atgtatccct actgttgctg tacc                                           144
<210> 4
   <211> 66
   <212> DNA
   <213> Hepatitis B virus
<400> 4
   caaggtatat tgcccgtttg tcctctaatt ccaggatctg caatcaacaa caggggacaa     60
   tgcaaa                                                                66
<210> 5
   <211> 120
   <212> DNA
   <213> Hepatitis B virus
<400> 5
   caaggtatat tgcccgtttg tcctctaatt ccaggatctg caatcaacaa caggggacaa     60
   tgcaaaacct gcacgactac tgctcacgga acctctatgt atccctactg ttgctgtacc    120
<210> 6
   <211> 15
   <212> DNA
   <213> Hepatitis B virus
<400> 6
   gcaatcaaca acagg 15
<210> 7
   <211> 21
   <212> DNA
   <213> Hepatitis B virus
<400> 7
   gcaatcaaca acaggggaca a 21
<210> 8
   <211> 27
   <212> DNA
   <213> Hepatitis B virus
<400> 8
   gcaatcaaca acaggggaca atgcaaa 27
<210> 9
   <211> 48
   <212> DNA
   <213> Hepatitis B virus
<400> 9
   gcaatcaaca acaggggaca atgcaaaacc tgcacgacta ctgctcac 48
<210> 10
   <211> 81
   <212> DNA
   <213> Hepatitis B virus
<400> 10
   gcaatcaaca acaggggaca atgcaaaacc tgcacgacta ctgctcacgg aacctctatg 60
   tatccctact gttgctgtac c 81
<210> 11
   <211> 60
   <212> DNA
   <213> Hepatitis B virus
<400> 11
   tgcaaaacct gcacgactac tgctcacgga acctctatgt atccctactg ttgctgtacc 60
<210> 12
   <211> 154
   <212> PRT
   <213> Hepatitis B virus
<400> 12
   Gly Gly Thr Thr Val Cys Leu Gly Gln Asn Ser Gln Ser Pro Thr Ser
   1 5 10 15
   Asn His Ser Pro Thr Ser Cys Pro Pro Thr Cys Pro Gly Tyr Arg Trp
   20 25 30
   Met Cys Leu Arg Arg Phe Ile Ile Phe Leu Phe Ile Leu Leu Leu Cys
   35 40 45
   Leu Ile Phe Leu Leu Ala Leu Leu Asp Tyr Gln Gly Ile Leu Pro Val
   50 55 60
   Cys Pro Leu Ile Pro Gly Ser Ala Ile Asn Asn Arg Gly Gln Cys Lys
   65 70 75 80
   Thr Cys Thr Thr Thr Ala His Gly Thr Ser Met Tyr Pro Tyr Cys Cys
   85 90 95
   Cys Thr Lys Pro Ser Asp Gly Asn Cys Thr Cys Ile Pro Ile Pro Ser
   100 105 110
   Ser Trp Ala Phe Gly Lys Phe Leu Trp Glu Trp Ala Ser Ala Arg Phe
   115 120 125
   Ser Trp Leu Ser Leu Leu Val Pro Phe Val Gln Trp Phe Val Gly Leu
   130 135 140
   Ser Pro Thr Val Trp Leu Ser Val Ile Trp
   145 150
<210> 13
   <211> 28
   <212> PRT
   <213> Hepatitis B virus
<400> 13
   Phe Leu Leu Ala Leu Leu Asp Tyr Gln Gly Ile Leu Pro Val Cys Pro
   1 5 10 15
   Leu Ile Pro Gly Ser Ala Ile Asn Asn Arg Gly Gln
   20 25
<210> 14
   <211> 48
   <212> PRT
   <213> Hepatitis B virus
<400> 14
   Phe Leu Leu Ala Leu Leu Asp Tyr Gln Gly Ile Leu Pro Val Cys Pro
   1               5                   10                  15
   Leu Ile Pro Gly Ser Ala Ile Asn Asn Arg Gly Gln Cys Lys Thr Cys
               20                  25                  30
   Thr Thr Thr Ala His Gly Thr Ser Met Tyr Pro Tyr Cys Cys Cys Thr
           35                  40                  45
<210> 15
   <211> 22
   <212> PRT
   <213> Hepatitis B virus
<400> 15
   Gln Gly Ile Leu Pro Val Cys Pro Leu Ile Pro Gly Ser Ala Ile Asn
   1               5                   10                  15
   Asn Arg Gly Gln Cys Lys
               20
<210> 16
   <211> 40
   <212> PRT
   <213> Hepatitis B virus
<400> 16
   Gln Gly Ile Leu Pro Val Cys Pro Leu Ile Pro Gly Ser Ala Ile Asn
   1               5                   10                  15
   Asn Arg Gly Gln Cys Lys Thr Cys Thr Thr Thr Ala His Gly Thr Ser
               20                  25                  30
   Met Tyr Pro Tyr Cys Cys Cys Thr
                       35                  40
<210> 17
   <211> 5
   <212> PRT
   <213> Hepatitis B virus
<400> 17
   Ala Ile Asn Asn Arg
   1               5
<210> 18
   <211> 5
   <212> PRT
   <213> Hepatitis B virus
<400> 18
   Ile Pro Gly Ser Ala
   1 5
<210> 19
   <211> 5
   <212> PRT
   <213> Hepatitis B virus
<400> 19
   Pro Gly Ser Ala Ile
   1 5
<210> 20
   <211> 5
   <212> PRT
   <213> Hepatitis B virus
<400> 20
   Gly Ser Ala Ile Asn
   1 5
<210> 21
   <211> 5
   <212> PRT
   <213> Hepatitis B virus
<400> 21
   Ser Ala Ile Asn Asn
   1 5
<210> 22
   <211> 5
   <212> PRT
   <213> Hepatitis B virus
<400> 22
   Ile Asn Asn Arg Gly
   1 5
<210> 23
   <211> 5
   <212> PRT
   <213> Hepatitis B virus
<400> 23
   Asn Asn Arg Gly Gln
   1 5
<210> 24
   <211> 5
   <212> PRT
   <213> Hepatitis B virus
<400> 24
   Asn Arg Gly Gln Cys
   1 5
<210> 25
   <211> 5
   <212> PRT
   <213> Hepatitis B virus
<400> 25
   Arg Gly Gln Cys Lys
   1 5
<210> 26
   <211> 7
   <212> PRT
   <213> Hepatitis B virus
<400> 26
   Ala Ile Asn Asn Arg Gly Gln
   1 5
<210> 27
   <211> 9
   <212> PRT
   <213> Hepatitis B virus
<400> 27
   Ala Ile Asn Asn Arg Gly Gln Cys Lys
   1 5
<210> 28
   <211> 16
   <212> PRT
   <213> Hepatitis B virus
<400> 28
   Ala Ile Asn Asn Arg Gly Gln Cys Lys Thr Cys Thr Thr Thr Ala His
   1 5 10 15
<210> 29
   <211> 27
   <212> PRT
   <213> Hepatitis B virus
<400> 29
   Ala Ile Asn Asn Arg Gly Gln Cys Lys Thr Cys Thr Thr Thr Ala His
   1               5                   10                  15
   Gly Thr Ser Met Tyr Pro Tyr Cys Cys Cys Thr
               20                  25
<210> 30
   <211> 20
   <212> PRT
   <213> Hepatitis B virus
<400> 30
   Cys Lys Thr Cys Thr Thr Thr Ala His Gly Thr Ser Met Tyr Pro Tyr
   1               5                   10                  15
   Cys Cys Cys Thr
               20
<210> 31
   <211> 22
   <212> DNA
   <213> Artificial Sequence
<220>
   <223> Primer 1
<400> 31
   gggtcaccat attcttggga ac 22
<210> 32
   <211> 26
   <212> DNA
   <213> Artificial Sequence
<220>
   <223> Primer 2
<400> 32
   tatacccaaa gacaaaagaa aattgg 26
<210> 33
   <211> 21
   <212> DNA
   <213> Artificial Sequence
<220>
   <223> Primer 3
<400> 33
   gactcgtggt ggacttctct c 21
<210> 34
   <211> 22
   <212> DNA
   <213> Artificial Sequence
<220>
   <223> Primer 4
<400> 34
   tacagacttg gcccccaata cc 22

## Beschreibung

Die Erfindung betrifft Sequenzen einer neuen Variante des Hepatitis B surface Antigens (HBsAg) und Methoden, diese Genom- und Protein-Variante sowie dagegen gerichtete Antikörper aus Patientenproben zu detektieren.

Die neuen Sequenzen führen zu 11 im Stand der Technik noch nicht bekannten Aminosäure-Austauschen in dem Hepatitis B surface Antigen (HBsAg) in den Aminosäurepositionen 96 bis 136 der Aminosäuresequenz des surface Antigens, wobei sich 10 Substitutionen in der Region der a-Determinante befinden (aa 101 bis aa 180)

Die Erfindung betrifft auch immunchemische Nachweisverfahren zum gleichzeitigen Nachweis dieser neuen HBV - Variante zusammen mit bekannten Varianten/Subtypen sowie die Verwendung der neuen Sequenzen in Verbindung mit bekannten Sequenzen zum gleichzeitigen Nachweis von HBV-spezifischen Antikörpern. Die Antigen bzw. Antikörper-Bestimmungen können jeweils in einem Testansatz differenzierend oder nicht-differenzierend durchgeführt werden.

Schließlich betrifft die Erfindung auch den Nachweis der entsprechenden Nukleinsäuren mit Hilfe sogenannter Nukleinsäure-Tests (z.B. Polymerase Chain Reaction, PCR) mit Hilfe geeigneter Primer sowie die Verwendung der neuen Aminosäuresequenzen zur Erzeugung von Impfstoffen.

Das Hepatitis B Virus ist bekanntermaßen der Auslöser einer Vielzahl von Erkrankungs-Verläufen von milden inapparenten Infektionen bis hin zu chronisch aktiven und fulminant verlaufenden durch virale Infektionen ausgelösten Leberentzündungen (Virushepatitiden).

Die chronische Infektion mit HBV stellt mit geschätzten 400 Millionen betroffenen Menschen ein globales Gesundheitsproblem dar (Lee, N. Engl. J. Med. 337; 1733-1745 (1997)).

Als geeignetste Prophylaxe für die weltweit häufig anzutreffende HBV-Infektion gilt die aktive Immunisierung (Stimulation der Antikörperantwort durch Antigengabe) und auch die passive Immunisierung (durch Injektion präformierter Antikörper).

Das HBV gehört zu den Hepadna-Viren und stellt ein Viruspartikel mit einem Durchmesser von 42 nm dar, das aus Kern und Hülle besteht. Das Genom des Virus ist eine doppelsträngige, ringförmige DNA- Sequenz von etwa 3200 Nukleotiden, die mindestens sechs verschiedene virale Gene kodieren (Tiollais et al., Nature 317: 489-495 (1985)).
Es liegen vier offene Leserahmen für die Bildung der viralen Proteine vor.

Im S-Gen liegt die Information für das HBV surface Antigen (HBsAg), das auch small protein (S) genannt wird. Daneben gibt es auch größere Formen, die als large protein (L) und middle protein (M) bezeichnet werden.

Allen drei Proteinen gemeinsam ist die 226 Aminosäuren umfassende S-HBsAg Sequenz (Gerlich et al., Viral Hepatitis and Liver Disease, Hollinger et al., William-Wilkens, Baltimore, MD, pages 121-134 (1991)).

Die Proteinregionen vor dem small HBs werden auch Pre-S1 und Pre-S2 bezeichnet, umfassen 108 bzw. 55 Aminosäuren und sind beide in dem L-Protein (389 Aminosäuren) enthalten, während das M-Protein nur das Pre-S2 zusammen mit dem S-Antigen umfasst (281 Aminosäuren). Die Pre-S Proteine weisen unterschiedliche Glykosilierungsgrade auf und tragen die Rezeptoren für die Erkennung der Leberzellen. Sofern nicht anders angegeben, beziehen sich die Aminosäurepositionen in dieser Anmeldung auf das S-Antigen (226 aa) ohne Pre-S1- und ohne Pre-S2-Region.

Das C-Gen trägt die Information für das Nukleokapsid Protein, Hepatitis B Core Antigen (HBcAg). Die Translation dieses Proteins kann bereits in der Pre-C-region starten und zur Bildung von Hepatitis B e-Antigen (HBeAg) führen. Das HBeAg weist gegenüber HBcAg eine andere Faltung und Immunogenität auf. HBeAg kommt im Gegensatz zu HBcAg frei im Serum vor und wird bei positivem Nachweis als Indikator für die Bildung von HBcAg und damit für die Bildung infektiöser Viruspartikel angesehen.

Die im Viruspartikel enthaltene Reverse Transkriptions DNA-Polymerase wird von P-Gen codiert und für das Transaktivator X-Gen wird eine ursächliche Rolle bei der Entstehung von HBV-assoziierten primären Leberzell-Karzinomen diskutiert.

Der virale Replikationszyklus von HBV umfasst eine intrazelluläre pre-genomische RNA, die im viralen Nukleocapsid in die DNA umgeschrieben wird. Da die HBV - eigene Reverse Transkriptase DNA- Polymerase über keine Richtigkeits-Lesefähigkeit verfügt (proof-reading capability), werden mit relativ hoher Häufigkeit falsche Nukleotide eingebaut. Als Folge weist das HBV eine Mutationsrate auf, die mit ca. 1 Nukleotid/10000 Basen/Infektionsjahr etwa dem 10-fachen dessen entspricht, was andere DNA Viren aufweisen (Blum, Digestion 56: 85-95 (1995); Okamoto et al., Jpn. J. Exp. Med. 57: 231-236 (1987)).
Daneben treten auch recht häufig Deletionen und Insertionen auf (Carman et al., Lancet 341: 349-353 (1993)).

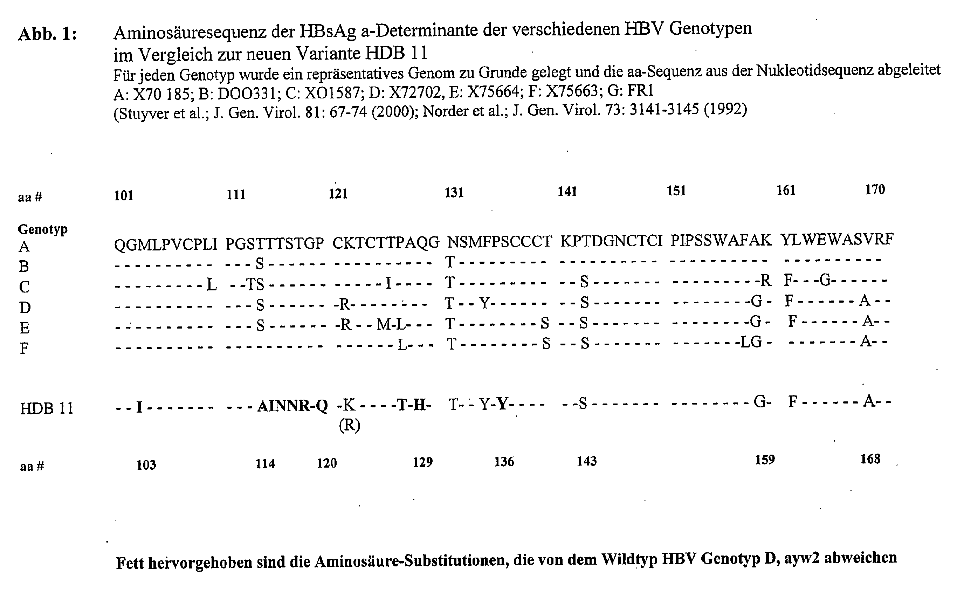

Die resultierende Variabilität von HBV drückt sich unter anderem in dem Auftreten von 9 serologisch definierten Subtypen (Courouce et al., Bibliotheca Haematologica 42: 1 (1976) und insgesamt mindestens 6 verschiedenen Genotypen aus, die mit A bis F bezeichnet werden (Abb. 1) und eine geographische Verteilung aufweisen. (Norder et al., J. Gen. Virol. 73: 3141-3145 (1992), Norder et al., Virology 198: 489-503 (1994)).
Außerdem werden eine Reihe von Mutanten beschrieben, bei denen 1 Aminosäure oder mehrere ausgetauscht vorliegen, fehlen oder überzählig sind.

Neben natürlicherweise stattfindenden Mutationen (Cooreman et al., Hepatology 30: 1287-1292 (1999) kann eine Gabe von HBV Immunglobulinen und / oder eine antivirale Therapie (z.B. mit Lamivudine) einen sogenannten Selektionsdruck ausüben, was zum vermehrten Auftreten sogenannter "Escape-Mutanten" führen und die Auftretens-Wahrscheinlichkeit von HBV-Mutanten deutlich erhöhen kann (Terrault et al., Hepatology 28: 555-561 (1998); Tillmann et al., Hepatology 30: 244-256 (1999); Hunt et al., Hepatology 31: 1037-1044 (2000).

Nicht alle HBV-Mutationen führen zu replikationsfähigen Viren und oft liegt eine Coexistenz von nicht vitalem und replikationsfähigem Virus vor, was auch die Sequenzierungs-Genauigkeit von isolierter DNA limitiert oder gar zur Nichterkennung von veränderten Sequenzen durch PCR, Klonierungsarbeiten mit anschließender Sequenzierung führt, wenn diese quantitativ < 10 % der Gesamt-DNA ausmachen (Cooreman et al., J. Biomed. Sci. 8: 237-247 (2001).

Demnach ist die Isolierung von Mutanten vorteilhaft, wobei die sich anschließende Identifizierung und Charakterisierung einzelner Mutanten möglicherweise zu verbesserten Vakzinen und Diagnostika führt.

Die Immunantwort nach einer Infektion mit HBV ist hauptsächlich gegen die sogenannte a-Determinante als eine allen Hepatitis B Viren gemeinsame Region des S-Proteins gerichtet, die sich auf der Oberfläche der Viruspartikel befindet (Gerlich et al., supra) und die den heterogensten Teil der B-Zell-Epitope des S-Gens darstellen.

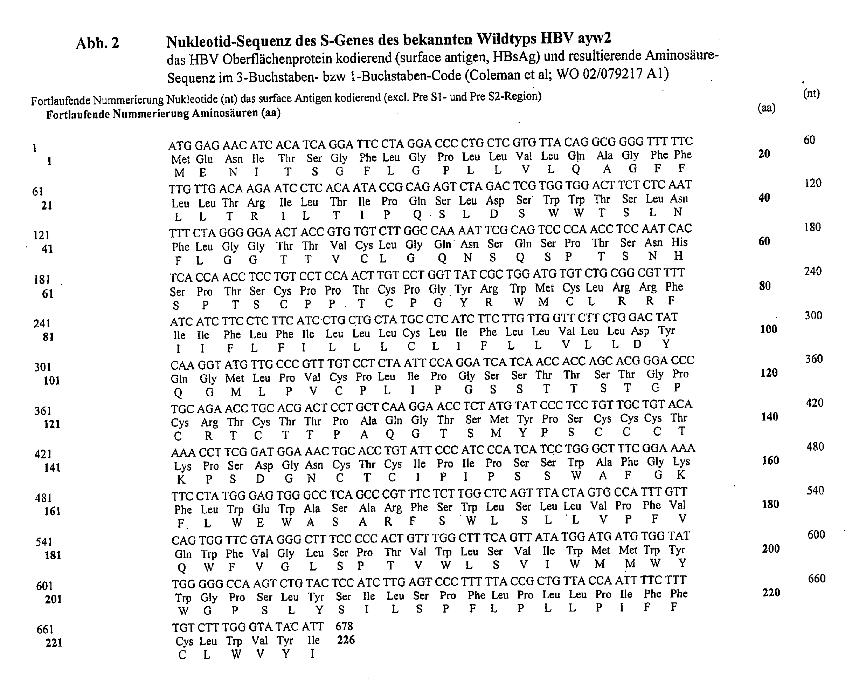

Als Bindestellen für Antikörper werden nach heutigem Kenntnisstand insgesamt mindestens 5 sich teilweise überlappende Epitope auf der a-Determinante zwischen Aminosäure-Position 101 und 180 angenommen (Abb. 1 und 2) wie durch die Anwendung von monoklonalen Antikörpern gezeigt werden konnte (Peterson et al., J. Immunol. 132: 920-927 (1984)).

Es handelt sich hauptsächlich um komplexe Konformations-Epitope, die durch mehrere Disulfid-Brücken stabilisiert werden. Teilweise liegen auch Sequenz-Epitope vor, die mit Hilfe synthetisch hergestellter zyklischer Peptid-Strukturen dargestellt werden können.

Sogenannte "schützende Antikörper", die nach einer natürlichen Infektion mit HBV im Serum zirkulieren, sind zu 99% gegen die sehr immunogene a-Determinante des HBV gerichtet (Jilg, Vaccine 16: 65-68 (1998).
Auf diese Tatsache stützt sich die breite Anwendung der Immunisierung mit Vakzinen, die entweder aus Humanserum isoliert oder gentechnologisch hergestellt wurden und die Verabreichung von Hepatitis B Immunglobulinen, die humane HBV - spezifische Antikörper enthalten. Beide prophylaktischen Strategien beruhen auf dem neutralisierenden Effekt, die HBs-spezifische Antikörper nach Bindung an die "a-Loop-Epitope" entfalten (Carman et al., Hepatology 24: 489-493 (1996), Muller et al., J. Hepatol. 13: 90-96 (1991) und Samuel et al., N. Engl. J. Med. 329: 1842-1847 (1993)).

Ähnlich beruhen die heute weit verbreiteten Diagnostika auf der Bindung von a-De-terminanten-spezifischen Antikörpern an Epitope der a-Determinante.
So wird bei der im Blutspendewesen weltweit angewendeten HBsAg-Bestimmung mit immunchemischen Bestimmungsmethoden im Serum von Spendern zirkulierendes HBV Oberflächenantigen mit Antikörpern gegen die a-Determinante (polyklonal oder monoklonalen Ursprungs) nachgewiesen und bei positivem Resultat die entsprechende Blutspende verworfen, um iatrogene HBV Infektionen durch HBV-kontaminiertes Blut zu verhindern. Eine weitere Anwendung der HBsAg-Bestimmung liegt im Nachweis einer vorliegenden akuten HBV-Infektion.

Umgekehrt wird mit der Bestimmung von HBs-spezifischen Antikörpern im Blut von Probanden mit einem positiven Bestimmungsresultat von HBsAg-spezifischen Antikörpern (Anti-HBs) nachgewiesen, dass entweder eine natürliche Infektion abgelaufen oder dass eine durchgeführte Vakzinierung erfolgreich verlaufen ist.

Schließlich beruht auch die Nukleinsäure-Testung z.B. mit Hilfe der Polymerase-Ketten-Reaktion ("Polymerase Chain Reaction" = PCR) auf der Verwendung von Primern, die für die HBV Nukleotide spezifisch sind.

Auf Grund der zentralen Rolle, die die a-Determinante bei der aktiven Immunisierung (Vakzinierung mit HBV Antigen), der passiven Immunisierung (Schutz durch HBV -spezische Immunglobuline), dem Nachweis von Impferfolg bzw. stattgefundener HBV Infektion (beides mittels Bestimmung von HBsAg-spezischen Antikörpern, Anti-HBs)und schließlich der Sicherheit im Blutspendewesen (HBsAg-Bestimmung und PCR), ist verständlich, dass in Fachkreisen das Auftreten von Mutanten und auch neuen Varianten mit großer Aufmerksamkeit verfolgt wird.

Als Konsequenz könnten in der a-Determinante des HBV veränderte aber replikationsfähige neue Mutanten und/oder Varianten sowohl das prophylaktische als auch das diagnostische Konzept unterlaufen (Brind et al., J. Hepatol. 26: 228-235 (1997), Fischer et al.,Transplant Proc. 31: 492-493 (1999), Ghany et al., Hepatology 27: 213-222 (1998), Protzer-Knolle et al., Hepatology 27: 254-263 (1998), Carman et al., Gastroenterology 102: 711-719 (1992) und Coleman et al., WO 02/079217 A1, (2002)).

Die Abgrenzung von Varianten und Mutanten des HBV ist nicht scharf, wobei ein diesbezüglicher Vorschlag breite Anwendung findet (Carman, J. Viral Hepat. 4 (suppl.1): 11-20 (1997). Dem zu Folge sollte die Bezeichnung "Variante" für natürlicherweise vorkommende Subtypen angewendet werden, die ohne bekannte Interferenz durch Selektionsdruck (antivirale Therapie und / oder Immunglobulin-Gabe) auftreten und ein geographisches Verteilungsmuster aufweisen.

Die Charakterisierung und anschließende Klassifizierung der Subtypen erfolgt mit Hilfe monoklonaler Antikörper und basiert auf einem veränderten Reaktionsmuster auf Grund des Austausches von einer oder wenigen Aminosäure(n). Die Grundlage der Klassifizierung stellen die Aminosäurepositionen 122 oder 160 der verbreitetsten HBV Sequenz dar: aa 122 und aa 160 = Lysin, K.

Alle Serotypen enthalten die Gruppen-spezifische a-Determinante, während die aa 122 und zusätzlich 133 und 134 den d- bzw. r-Subtyp und aa 160 die Zugehörigkeit zum w- bzw r-Subtyp bestimmen. Auf dieser Basis lassen sich HBV Subtypen grob in adr, adw, ayr oder ayw einteilen, die sich weiter in mindestens 9 Sub-Subtypen unterscheiden lassen: ayw1, ayw2, ayw3, ayw4, ayr, adwr2, adw4, adrq+ und adrq- (Swenson et al., J. Virol. Meth. 33: 27-28 (1991), Blitz et al. J. Clin. Microbiol. 36: 648-651, Ashton-Rickardt et al., J. Med. Virol. 29: 204-214 (1989)).

Da diese Klassifizierung eine serologische Reaktivität zur Grundlage macht, muß nicht jede Typisierung notwendigerweise eine Variabilität auf der Aminosäure-Ebene bedeuten, weshalb dem Genotyping auf der S-Gen-Ebene der Vorzug gegeben wird (Ohba et al., Virus Res. 39: 25-34 (1995).

Subtypen treten aus noch nicht bekannten Gründen in bestimmten geographischen und ethnischen Mustern auf.

Die Bezeichnung Mutation sollte nach Carman Varianten vorbehalten bleiben, die ausschließlich unter Selektionsdruck wie Vakzinierung oder antivirale Therapie entstehen. Es sind bereits viele Mutationen beschrieben worden, von denen manche zu diagnostisch falschen Befundungen führten (Carman et al., Lancet 345: 1406-1407) und von denen die nachstehend genannten aa-Austausche beispielhaft zitiert werden:

| Consensus: | aa-Position | Mutante: |
|---|---|---|
| I | 110 | V |
| P | 111 | T |
| T | 114 | S |
| T | 116 | S |
| P | 120 | T/S |
| T | 123 | A/N |
| I/T | 126 | A/S |
| Q | 129 | H/R |
| K/M | 133 | L |
| T | 143 | M/L |
| D | 144 | H/A/E |
| G | 145 | R/A |
| A | 157 | R |

sowie Cystein Austausche in den aa-Positionen 107, 124, 137, 147 & 149.

(Coleman, supra; Okamoto et al., Pediatr. Res. 32: 264-268 (1992); Zhang et al., Scand. J. Infect. Dis. 28: 9-15 (1996); Zuckermann et al., Lancet 343: 737-738 (1994)).

Überraschend wurde in einer Humanprobe eines an Leberentzündung erkrankten ägyptischen Patienten (interne Nummer: 118234, Entnahme vom 02. Okt. 2002) ein atypisches Reaktionsmuster von Hepatitis-Markern gefunden.

Neben dem Krankheitsbild mit Erhöhung der für eine derartige Infektion typischen Leberwerte weisen auch nachgewiesene Hepatitis Core Antikörper der IgM-Klasse auf eine akute HBV-Infektion hin, ohne dass allerdings der HBsAg-Nachweis mit einem zugelassenen leistungsstarken HBsAg-ELISA gelang.

Eine durchgeführte PCR ergab überraschend ein positives Resultat und das Sequenzierungsergebnis führte völlig überraschend zu den in Abb. 3 und 4 dargestellten Nukleotid-Sequenzen und den in Abb. 5 und 6 abgebildeten Aminosäure-Sequenzen.

Aus diesen Sequenzen wird deutlich, dass es sich völlig überraschend nicht um eine Punktmutation, d.h. Austausch weniger Nukleotide und auch nicht um einen möglicherweise serologisch zu charakterisierenden Subtyp handelt, da insgesamt n=11 Aminosäuren in der Region von aa 96 bis 180 gegenüber dem Genotyp D substituiert vorliegen. Im Hinblick auf die Häufigkeit der Aminosäure-Substitutionen ist völlig überraschend davon auszugehen, dass es sich um einen neuen Wildtyp handelt oder dass die Mutationen so ausgeprägt sind, dass die Konsequenz eher als neue Variante zu beschreiben ist, die im Folgenden als HDB 11-Variante bezeichnet wird.

Die Analyse der besten Übereinstimmung der Aminosäuresequenz der a-Determinante mit bekannten Sequenzen verweist auf Genotyp D (Abb. 1), Subtyp ayw2 (Abb. 2) von dem sich die neue Variante überraschend allerdings noch in 11 aa-Positionen unterscheidet. Prominentestes Merkmal sind die 10 Substitutionen in der Region wischen aa 103 und 136 gemäß Abb. 1, 5 und 6.

Selbst wenn man die Möglichkeit berücksichtigt, dass eine Koexistenz der neuen HDB 11-Variante mit einem bekannten Wildtyp vorliegt, bleibt als überraschendes Merkmal die charakteristische und neue Sequenz in der Aminosäure-Region aa 114 bis 120 bestehen, die mit 6 neuen Aminosäure-Sequenzen/ -Substitutionen zu beschreiben ist (Abb. 1).

Die vorliegende Erfindung betrifft die in den Ansprüchen beschriebenen Oligo- oder Polypeptide. Die in SEQ ID NO:14 gezeigte Aminosäuresequenz entspricht den Aminosäurepositionen 93 bis 140 des S-Antigens von Hepatitis B, Welches insgesamt eine Länge von 226 Aminosäuren aufweist.

Die in SEQ ID NO:12 gezeigte Aminosäuresequenz entspricht den Aminosäurepositionen 43 bis 196 des S-Antigens des Hepatitis B-Virus, welches eine Länge von 226 Aminosäuren aufweist.

Die Bestimmung der Identität zwischen zwei Aminosäuresequenzen ist dem Fachmann an sich bekannt und kann mit üblichen Computerprogrammen durchgeführt werden. Vorzugsweise wird die Bestimmung der Identität mit dem Computerprogramm "Bestfit" der Genetics Computer Group (Madison, WI) durchgeführt. Die Parameter werden in den Standardeinstellungen (default) verwendet. Vorzugsweise wird die am Prioritätstag der vorliegenden Anmeldung aktuelle Programmversion verwendet. Ein hoher Prozentwert der Identität bedeutet eine hohe Entsprechung, Gleichheit oder Äquivalenz zweier Sequenzen.

Die Mindestlänge der erfindungsgemäßen Oligo- oder Polypeptide beträgt 7, vorzugsweise 8 Aminosäuren. Die Gesamtlänge des Oligo- oder Polypeptids ist in der Regel 7 bis 300 Aminosäuren, vorzugsweise 8 bis 200 Aminosäuren. Die Oligo- oder Polypeptide können auch Fremdaminosäuren enthalten, die nicht vom Genom eines Hepatitis B-Virus kodiert werden. So können Aminosäuren enthalten sein, die die Kopplung an feste Phasen erleichtern oder die Kopplung an Markierungssubstanzen ermöglichen. Es können Aminosäuren enthalten sein, die aufgrund der Klonierung entstanden sind und bei der rekombinanten Expression mit exprimiert wurden. Schließlich kann das erfindungsgemäße Oligo- oder Polypeptid ein Fusionsprotein sein, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner sind dem Fachmann an sich bekannt.

In einer anderen Ausführungsform enthalten die Oligo- oder. Polypeptide keine Fremdaminosäuren, die nicht vom Genom eines HBV kodiert werden. Entsprechend bestehen diese Oligo- oder Polypeptide aus einer der oben und/oder in den Ansprüchen beschriebenen Aminosäuresequenzen.

Das erfindungsgemäße Oligo- oder Polypeptid ist vorzugsweise immunogen, d.h. es kann eine Antikörperantwort in einem Säugerorganismus induzieren. Üblicherweise enthält das Oligo- oder Polypeptid wenigstens eine antigene Determinante oder wenigstens ein Epitop. In einer besonderen Ausführungsform enthält das Oligo- oder Polypeptid ein Epitop, das in anderen HBV-Varianten, z. B. in Subtyp ayw22, nicht enthalten ist.

Vorzugsweise umfasst das Oligo- oder Polypeptid eine der Aminosäuresequenzen SEQ ID NO:12, SEQ ID NO: 13, SEQ ID NO: 14, SEQ ID NO:15, SEQ ID NO:16, SEQ ID NO:26, SEQ ID NO:27, SEQ ID NO:28 und, SEQ ID NO:29.

Ein weiterer Aspekt der Erfindung ist ein immunogenes Peptid oder eine Mischung immunogener Peptide, enthaltend eines oder mehrere der in den Ansprüchen beschriebenen Oligo- oder Polypeptide. Das immunogene Peptid oder die immunogene Mischung kann das/die Oligo- oder Polypeptide alleine oder in Verbindung mit bekannten HBV-Immunogenen enthalten.

Gegenstand der vorliegenden Erfindung sind auch die in den Ansprüchen beschriebenen Nukteinsäuremoleküle.

Die Nukleotidsequenz SEQ ID NO:3 kodiert für die Aminosäuresequenz SEQ ID NO:14.

Die Nukleotidsequenz SEQ ID NO:1 kodiert für die Aminosäuresequenz SEQ ID NO:12.

Identität ist definiert als Grad der Gleichheit zwischen zwei Strängen von zwei DNA Segmenten. Ausgedrückt wird die Identität als prozentualer Wert, indem die Anzahl identischer Basen zweier zu vergleichender Sequenzen geteilt durch die Länge der kürzeren Sequenz und mit 100 multipliziert wird (Smith et al., Adv. Appl. Mathem. 2: 482-489 (1981).

Vorzugsweise wird die Bestimmung der Identität mit dem Computerprogramm "Bestfit" der Genetics Computer Group (Madison, WI) durchgeführt. Die Parameter werden in den Standardeinstellungen (default) verwendet. Vorzugsweise wird die am Prioritätstag der vorliegenden Anmeldung aktuelle Programmversion verwendet. Ein hoher Prozehtwert der Identität bedeutet eine hohe Entsprechung, Gleichheit oder Äquivalenz zweier Sequenzen.

Diese Bewertung ist auch auf Aminosäuresequenzen von Peptiden und Proteinen anwendbar (Dayhoff, Atlas of Protein Sequences and Structure, M.O Dayhoff ed. 5 Suppl. 3: 353-358, Nat. Biom. Res. Found., Washington D.C., USA, Gribskov, Nucl. Acids Res. 14 (6): 6745-66763 (1986).

Verfahren zur Bestimmung, ob eine gegebenes Oligo- oder Polynukleotid mit einem anderen Polynukleotid hybridisiert, sind dem Fachmann an sich bekannt. Ein spezielles Beispiel für "stringente Bedingungen" sind folgende Bedingungen: a) 16-stündige Inkubation bei 42 °C in einer Lösung enthaltend 50 % Formamid, 5×SSC (150 mM NaCl, 15 mM Trinatriumcitrat), 50 mM Natriumphosphat pH 7,6, 5×Denhardt's Lösung, 10% Dextransulfat und 20 µg/ml denaturierte, gescherte Lachssperma-DNA; b) anschließend Waschen in 0,1×SSC bei ungefähr 65 °C. Hybridisierungs- und Waschbedingungen sind dem Fachmann an sich bekannt und beispielhaft in Sambrook et al., Molecular Cloning: A Laboratory Manual, Second Edition, Cold Spring Harbor, N.Y., (1989) angegeben. Eine Nukleotidsequenz hybridisiert spezifisch an ein gegebenes Polynukleotid, wenn sie nicht oder wesentlich schwächer an andere Nukleotidsequenzen hybridisiert. Im vorliegenden Fall kann das bedeuten, dass die Nukleotidsequenz nicht oder nur schwach an für HBsAg kodierende Polynukleotide aus herkömmlichen HBV-Varianten (z. B. Genotyp D, Subtyp ayw2) hybridisiert.

Die Erfindung betrifft auch ein Oligo- oder Polynukleotid umfassend eine Nukleotidsequenz, die für ein erfindungsgemäßes Oligo- oder Polypeptid kodiert, wie es in dieser Anmeldung beschrieben ist. Ein weiterer Aspekt der Erfindung ist ein Oligo- oder Polynukleotid umfassend eine zu den oben beschriebenen Nukleotidsequenzen komplementäre Nukleotidsequenz.

Die Oligo- oder Polynukleotide können auch Nukleotide enthalten, die nicht vom Genom eines Hepatitis B-Virus herstammen. So können Nukleotide enthalten sein, die für bestimmte Aminosäuren kodierten, die gewünschte Funktionen erfüllen sollen, wie oben beschrieben. Es können Nukleotide enthalten sein, die aufgrund der Klonierung entstanden sind, z. B. um bestimmte Schnittstellen einzuführen. Schließlich kann das erfindungsgemäße Oligo- oder Polynukleotid für ein Fusionsprotein kodieren, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner und die sie kodierende DNA sind dem Fachmann an sich bekannt.

Bevorzugte Oligo- oder Polynukleotide der vorliegenden Erfindung umfassen eine Nukleotidsequenz, die ausgewählt ist aus der Gruppe bestehend aus SEQ ID NO:1, SEQ ID NO:2, SEQ ID NO:3, SEQ ID NO:4, SEQ ID NO:5, SEQ ID NO:7, SEQ ID NO:8, SEQ ID NO:9, und SEQ ID NO:10.

Die erfindungsgemäßen Polynukleotide können auch markiert sein, beispielsweise durch eine Fluoreszenzmarkierung oder eine radioaktive Markierung. Derartige Polynukleotide können vorteilhaft in einer Hybridisierungsreaktion oder einer Polymerasekettenreaktion (PCR) eingesetzt werden.

Die Erfindung betrifft auch einen Vektor oder ein Plasmid enthaltend ein Oligo- oder Polynukleotid gemäß der Erfindung. Das Plasmid kann beispielsweise ein Klonierungsvektor sein, der dazu dient, die Nukleinsäure in Wirtszellen zu vermehren oder bestimmte Restriktionsschnittstellen zur Verfügung zu stellen. Expressionsvektoren sind Vektoren, die die Expression der klonierten Nukleinsäure in Wirtszellen erlauben. Wirtszellen können verschiedene prokaryontische oder eukaryontische Zellen sein. Prokaryontische Wirtszellen sind z. B. bakterielle Zellen wie *E*. *coli*-Zellen. Die erfindungsgemäßen Expressionsvektoren können bestimmte Steuerelemente wie z.B. Promotoren oder Bindungsstellen für Repressionsfaktoren enthalten. In einer weiteren Ausführungsform enthalten die Expressionsvektoren einen Nukleinsäureabschnitt, der für einen Teil eines Fusionsproteins kodiert.

Die Erfindung betrifft ebenfalls eine Zelle, z. B. eine Wirtszelle, die ein erfindungsgemäßes Polynukleotid, Plasmid oder einen erfindungsgemäßen Vektor enthält. Die Wirtszellen können unter geeigneten Bedingungen kultiviert werden, so dass eine Transkription der enthaltenen Nukleinsäure und nachfolgende Translation stattfindet. Die Erfindung betrifft auch ein Verfahren zur Herstellung eines Polypeptids, bei dem man ein Polynukleotid, ein Plasmid oder einen Expressionsvektor der Erfindung in Wirtszellen einbringt und die Wirtszellen unter Bedingungen kultiviert, die zur Expression des Polypeptids führen. Gegebenenfalls kann anschließend das Polypeptid aus den Wirtszellen gewonnen werden. Vorzugsweise findet die Herstellung des Polypeptids in Bakterien, am bevorzugtesten in E. *coli*-Zellen statt. Geeignete Mittel und Bedingungen zur Kultivierung sind beispielsweise in Ausubel et al. (1993) "Current Protocols in Molecular Biology" beschrieben. Die Gewinnung des exprimierten Polypeptids geschieht nach für den Fachmann an sich bekannten Methoden. Verschiedene Verfahren zur Proteinreinigung sind beispielsweise beschrieben in Scopes R. (1994) "Protein Purification: Principles and Practice" (3rd edition) Springer Verlag.

Die Polypeptide und Peptide der vorliegenden Erfindung können jedoch auch chemisch durch bekannte Verfahren wie z.B. Festphasensynthese hergestellt werden. Ebenso können die erfindungsgemäßen Polynukleotide durch bekannte Verfahren der chemischen Synthese hergestellt werden. Durch chemische Synthese erhaltene Polynukleotidfragmente können dann auch enzymatisch durch Ligasen verknüpft werden. Die erfindungsgemäßen Oligo-oder Polynukleotide können auch durch "site-directed mutagenesis" aus bekannten Sequenzen hergestellt werden, indem an bestimmten Positionen Punktmutationen eingeführt werden. Derartige Verfahren sind dem Fachmann an sich bekannt.

Ein weiterer Aspekt der Erfindung ist ein Antikörper, der an ein erfindungsgemäßes Oligo-oder Polypeptid bindet. Solche Antikörper können auf bekannte Weise hergestellt werden, entweder mittels eines Oligo- oder Polypeptids der Erfindung, z. B. eines Peptids mit einer der Sequenzen SEQ ID NO:12 bis 30, oder eines Fragments davon (Harlow und Lane (1988) Antibodies: A Laboratory Manual; Cold Spring Harbor Laboratory). Es kann sich um polyklonale oder monoklonale Antikörper handeln, monoklonale Antikörper sind aber bevorzugt. Vorzugsweise handelt es sich um spezifische Antikörper, die gegen das HBsAg der neuen HBV-Variante gerichtet sind, die aber HBsAg aus anderen HBV-Varianten, z. B. Genotyp D Subtyp ayw2, nicht erkennen. Solche Antikörper können erhalten werden, indem aufgrund eines Sequenzvergleichs der Aminosäuresequenzen des neuen HBsAg und HBsAg aus bekannten Stämmen Peptide ermittelt werden, die spezifisch für das neue HBsAg sind, und diese Peptide zur Herstellung der Antikörper verwendet werden. Es ist auch möglich, eine Mischung polyklonaler Antikörper herzustellen und sie durch Inkubation mit bekanntem HBsAg zu depletieren. In einer anderen Ausführungsform erkennt der Antikörper nicht nur das neue HBsAg, sondern auch bekannte HBsAg-Varianten. Dadurch ist es möglich, gleichzeitig verschiedene Varianten von HBsAg zu detektieren.

Der Antikörper der Erfindung kann an ein Oligo- oder Polypeptid binden, das aus einer Aminosäuresequenz besteht, die ausgewählt ist aus der Gruppe bestehend aus SEQ ID NO:12, SEQ ID NO:13, SEQ ID NO:14, SEQ ID NO:15, SEQ ID NO:16, SEQ ID NO: 17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29 und SEQ ID NO:30. Besonders bevorzugt bindet der Antikörper an ein Oligo- oder Polypeptid, das aus einer Aminosäuresequenz besteht, die ausgewählt ist aus der Gruppe bestehend aus SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29 und SEQ ID NO:30. In einer besonderen Ausführungsform bindet der Antikörper nicht an die a-Determinanten der bekannten HBV-Genotypen A, B, C, D, E und F (siehe Abb. 1). In einer besonderen Ausführungsform bindet der Antikörper nicht an die a-Determinante von HBV Genotyp D, Subtyp ayw2.

Die Erfindung betrifft auch einen Testkit zum Nachweis von Hepatitis B-Viren, enthaltend ein erfindungsgemßes Oligo- oder Polypeptid, ein erfindungsgemäßes Oligo- öder Polynukleotid und/oder einen erfindungsgemäßen Antikörper.

Die Erfindung bezieht sich auch auf ein immunogenes Peptid oder eine Mischung immunogener Peptide, enthaltend ein oder mehrere erfindungsgemäßen Oligo- oder Polypeptide alleine oder in Verbindung mit bekannten HBV-Immunogenen.

Ein weiterer Aspekt der Erfindung ist ein Verfahren zum Nachweis eines Hepatitis B-Antigens, dadurch gekennzeichnet, dass (a) eine Probe mit einem erfindungsgemäßen Antikörper unter Bedingungen inkubiert wird, die die Bildung eines Antigen-Antikörper-Komplexes gestatten; und (b) ein Antigen-Antikörper-Komplex, der den Antikörper enthält, nachgewiesen wird.

Es können monoklonale oder polyklonale Antikörper (oder Mischungen bzw. Fragmente davon oder Mischungen von Fragmenten), die mit Epitopen der neuen HBV Variante reagieren, dazu verwendet werden, die a-Determinante der erfindungsgemäßen HBV Variante in Form der gesamten Polypeptidsequenz oder Teilen davon in Untersuchungsproben zu bestimmen: HBsAg der HDB 11-Variante.

Dem Fachmann ist eine Vielzahl von Bestimmungsmethoden geläufig, bei denen mit einem oder mehreren monoklonalen Antikörper(n) oder polyklonalen Antikörpern (oder Mischungen davon bzw. Fragmenten oder Mischungen von Fragmenten), die spezifisch für die a-Determinante der HBV Variante sind, Immunkomplexe gebildet oder deren Bildung gehemmt werden.

Eine spezielle Ausführung stellt der sogenannte Enzymimmunoassay dar, von dem ein mögliches. Testprinzip im folgenden beispielhaft beschrieben wird, ohne jedoch die erfindungsgemäße Idee darauf zu beschränken:

Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Antikörper oder Fragmente davon mit der Untersuchungsprobe inkubiert. An die Antikörper gebundenes HBsAg wird nach Entfernen überschüssiger Probe detektiert, indem eine weitere Inkubation erfolgt mit Anti-HBs-Antikörpern (monoklonal oder polyklonal oder Fragmente bzw. Mischungen dieser Fragmente), die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen HBsAg-Gehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

All diese Ausführungsformen entsprechen dem Stand der Technik, so dass unter "Bestimmung von HBsAg der neuen HBV Variante" mit der vorliegenden Erfindung alle Verfahren verstanden werden, die sich zum Nachweis von Polypeptidsequenzen oder Antigenen der neuen HBV Variante eignen, ungeachtet ob alleine das HBsAg der neuen Variante bestimmt wird oder in Verbindung mit HBsAg von bekannten a-Determinanten und/oder bekannten Mutationen in der a-Region.

Ebenso ist es möglich, aus ökonomischen Gründen eine HBsAg-Bestimmung mit einer Nachweismethode gegen einen weiteren Analyten (z.B. HIV-Antigen oder die gleichzeitige Bestimmung von HBV Varianten-HBsAg und dagegen gerichtete spezifische Antikörper) in einem Testansatz (differenzierend oder nicht-differenzierend) zu kombinieren.

Die Erfindung betrifft auch ein Verfahren zum Nachweis von Antikörpern, die gegen ein Hepatitis B-Antigen gerichtet sind, dadurch gekennzeichnet, dass (a) eine Probe mit einem erfindungsgemäßen Oligo- oder Polypeptid unter Bedingungen inkubiert wird, die die Bildung eines Antigen-Antikörper-Komplexes gestatten; und (b) der Antikörper-Antigen-Komplex, der das Oligo- oder Polypeptid enthält, nachgewiesen wird.

Eine spezielle Ausführung stellt der sogenannte Enzymimmunoassay dar, von dem ein mögliches Testprinzip im folgenden beispielhaft beschrieben wird, ohne jedoch die erfindungsgemäße Idee darauf einzuschränken:

Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Epitop-tragende Polypeptid- oder Protein-Sequenzen mit der Untersuchungsprobe inkubiert. An die Epitope gebundene Antikörper werden nach Entfernen überschüssiger Probe detektiert, indem eine weitere Inkubation erfolgt mit Epitop-tragenden Polypeptid- oder Protein-Sequenzen, die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen Antikörpergehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

Ebenso können durch Wahl eines geeigneten Testprinzips auch Variantenspezifische monoklonale oder polyklonale Antikörper zur Bestimmung von Anti-HBs-Antikörpern (z.B. in einem kompetitiven Testformat) herangezogen werden. Es ist ebenso bekannt, dass durch Wahl des Testprinzips auch eine Differenzierung der Immunglobulin-Klassen erfolgen kann (z.B. durch das "indirekte" Verfahren mit einem zweiten Klassenspezifischen Antikörper (z.B. IgG oder IgM spezifisch) mit Sonde oder mit Hilfe des sogenannten Anti-µ-Prinzips (IgM spezifisch). Natürlich müssen die Methoden und Materialen (inkl. Sonde und Polypeptidsequenzen) dem jeweiligen Ziel angepaßt werden.

All diese Ausführungsformen entsprechen dem Stand der Technik, so dass unter "Bestimmung von Antikörpern, die für die a-Determinante der neuen HDB 11-Variante spezifisch sind" mit der vorliegenden Erfindung alle Verfahren verstanden werden, die sich zum Nachweis von Immunglobulinen und/oder Immunglobulin-Klassen gegen die neue HBV Variante eignen, ungeachtet ob alleine der Antikörper gegen die neue Variante gesucht wird oder in Verbindung mit Antikörpern gegen bekannte a-Determinanten und / oder bekannte Mutationen in der a-Region.

In einem anderen Verfahren kann eine Hepatitis B-Nukleinsäure nachgewiesen werden. Dieses Verfahren ist dadurch gekennzeichnet, dass (a) eine Probe mit einem erfindungsgemäßen Oligo- oder Polynukleotid unter Bedingungen inkubiert wird, die die selektive Hybridisierung des Oligo- oder Polynukleotids mit einer Hepatitis B-Nukleinsäure in der Probe gestatten; und (b) bestimmt wird, ob Polynukleotidduplexe gebildet wurden, die das Oligo- oder Polynukleotid umfassen.

Die Hepatitis B-Nukleinsäure kann auch dadurch nachgewiesen werden, dass (a) eine Probe mit wenigstens einem erfindungsgemäßen Oligo- oder Polynukleotid unter Bedingungen inkubiert wird, die die selektive Hybridisierung des Oligo- oder Polynukleotids mit einer Hepatitis B-Nukleinsäure in der Probe gestatten; (b) eine Polymerasekettenreaktion durchgeführt wird; und (c) bestimmt wird, ob eine Nukleinsäure amplifiziert wurde.

Die Erfindung betrifft auch die Verwendung eines erfindungsgemäßen Oligo- oder Polynukleotids als Primer und/oder als Sonde. Die vorliegenden Nukleotidsequenzen können benutzt werden, um Primer und/oder Gensonden herzustellen, weshalb auch Kits, enthaltend Primer und/oder Sonden zum Nachweis von HBV Varianten-spezifischer Nukleinsäure entweder alleine oder in Verbindung mit bekannten HBV Nukleotidsequenzen in Untersuchungsproben ebenfalls Gegenstand der Erfindung sind.

Auf Basis der vorliegenden Nukleotidsequenzen können Primer entwickelt werden, die in der sog. "Polymerase Chain Reaction" (PCR) Verwendung finden. Die PCR stellt eine Methode dar, um eine gewünschte Nukleotidsequenz einer Nukleinsäure oder eines Nukleinsäuregemisches zu amplifizieren. Dabei werden die Primer jeweils spezifisch durch eine Polymerase mit der gewünschten Nukleinsäure als Leseraster verlängert. Nach Dissoziation von dem Originalstrang werden neue Primer hybridisiert und wiederum durch die Polymerase verlängert. Durch Wiederholung dieser Zyklen wird eine Anreicherung der gesuchten Zielsequenz-Moleküle erreicht.

In Bezug auf Nukleinsäuretests (NAT) ist es möglich, Nukleotid-Sequenzen der vorliegenden Erfindung zu verwenden, um DNA- Oligomere von 6-8 Nukleotiden oder größer herzustellen, die geeignet sind, als Hybridisierungssonden das virale Genom der hier beschriebenen HBV Variante in Personen nachzuweisen, die möglicherweise die Virus-Variante tragen, oder z.B. im Blutspendewesen Blutkonserven auf Vorhandensein des Varianten-Genoms entweder gezielt oder in Kombination mit dem Nachweis von Nukleotidsequenzen bekannter HBV Varianten und/oder HBV-Mutatanten zu screenen.

Ebenso können auf Basis der gefundenen Nukleotidsequenzen der neuen HBV Variante entsprechende Primer entwickelt werden, die für die neue Variante spezifisch sind oder sowohl die neue Variante als auch im Stand der Technik bekannte Varianten detektieren können.

Die Erfindung betrifft auch die Verwendung eines erfindungsgemäßen Oligo- oder Polynukleotids oder eines erfindungsgemäßen Oligo- oder Polypeptids zur Herstellung eines Arzneimittels zur Behandlung oder Vorbeugung einer HBV-Infektion. Insbesondere können die erfindungsgemäßen Oligo- oder Polynukleotide bzw. Oligo- oder Polypeptide zur Herstellung eines Impfstoffs gegen HBV verwendet werden.

Ferner schließt die Erfindung auch eine Vakzine ein umfassend ein Polypeptid der vorliegenden Erfindung und ein gebräuchliches Adjuvans (z.B. Freund sches adjuvans, Phosphat-gepufferte Saline o.ä.). Eine derartige Vakzine kann dazu verwendet werden, um die Bildung von Antikörpern in Säugetieren anzuregen.

Die Nukleotidsequenzen der Erfindung können auch dazu verwendet werden, sog. Antisense Oligonukleotide darzustellen (gegebenenfalls für therapeutische Zwecke).

Weitere Aspekte der vorliegenden Erfindung sind die folgenden Gegenstände (1) bis (16):
(1) Ein isoliertes Oligo- oder Polynukleotid mit einer der Sequenzen ausgewählt aus der Gruppe von SEQ ID NO:1 bis SEQ ID NO:11, sofern dieses in den Ansprüchen beschrieben ist:
   SEQ ID NO:1
   SEQ ID NO:2 SEQ ID NO:3
   SEQ ID NO:4 SEQ ID NO:5
   **SEQ ID NO:6**
      340 GCAATCAACAACAGG **354**
   **SEQ ID NO:7**
      **340** GCAATCAACAACAGGGGACAA **360**
   **SEQ ID NO:8**
      **340** GCAATCAACAACAGGGGACAATGCAAA **366**
   **SEQ ID NO:9**
      **340** GCAATCAACAACAGGGGACAATGCAAAACCTGCACGACTACTGCTCAC 387
   SEQ ID NO:10
   **SEQ ID NO:11**
(2) Ein isoliertes Oligo- oder Polynukleotid, welches für HBs-Antigen des Hepatitis B-Virus kodiert und ein Oligo- oder Polynukleotid nach (1) enthält.
(3) Ein Fragment eines Oligo- oder Polynukleotids, welches für HBs-Antigen des Hepatitis B-Virus kodiert, dadurch gekennzeichnet, dass das Fragment ein Oligo- oder Polypeptid nach (1) enthält.
(4) Ein isoliertes Oligo- oder Polynukleotid, welches für die a-Determinante des HBs-Antigens des Hepatitis B-Virus kodiert und ein Oligo- oder Polynukleotid nach (1) enthält.
(5) Ein Primer, der für ein Oligo- oder Polynukleotid gemäß einem der Gegenstände (1) bis (4) spezifisch ist.
(6) Ein Vektor, der mindestens ein Oligo- oder Polynukleotid gemäß einem der Gegenstände (1) bis (3) enthält.
(7) Eine Wirtszelle, die einen Vektor nach (6) enthält.
(8) Ein Oligo- oder Polypeptid weiches durch ein Oligo- oder Polynukleotid nach einem der Gegenstände (1) bis (3) kodiert wird.
(9) Isoliertes Oligo- oder Polypeptid, das eine Aminosäuresequenz ausgewählt aus der Gruppe von SEQ ID NO:12 bis SEQ ID NO:30 hat, sofern dieses in den Ansprüchen beschrieben ist:
   SEQ ID NO: 12
   SEQ ID NO:13 SEQ ID NO:14
   SEQ ID NO:15
   SEQ ID NO:16
   **SEQ ID NO:17**
      **114** A I N N R **118**
   **SEQ ID NO:18**
      **110** I P G S A 114
   **SEQ ID NO: 19**
      **111** P G S A I **115**
   **SEQ ID NO:20**
      **112** G S A I N **116**
   **SEQ ID No.: 21**
      113 S A I N N 117
   SEQ ID NO:22
      115 I N N R G **119**
   **SEQ ID NO:23**
      **116** N N R G Q **120**
   **SEQ ID NO:24**
      **117** N R G Q C **121**
   **SEQ ID NO:25**
      **118** R G Q C K **122**
   **SEQ ID NO:26**
      **114** A I N N R G Q **120**
   **SEQ ID NO:27**
      **114** A I N N R G Q C K **122**
   **SEQ.ID NO:28**
      **114** A I N N R G Q C K T C T T T A H **129**
   **SEQ ID NO:29**
   **SEQ ID NO:30**
      **121** C K T C T T T A H G T S M Y P Y C C C T **140.**
(10) Isoliertes Polypeptid entsprechend der Sequenz des HBs-Antigens des Hepatitis B-Virus, dadurch gekennzeichnet, dass es ein Oligo- oder Polypeptid gemäß einem der Gegenstände (8) enthält.
(11) Fragment eines Polypeptids, welches der Sequenz des HBs-Antigens des Hepatitis B-Virus entspricht, dadurch gekennzeichnet, dass das Fragment ein Oligo- oder Polypeptid gemäß einem der Gegenstände (8) bis (9) enthält.
(12) Isoliertes Polypeptid, welches für die a-Determinante des HBs-Antigens des Hepatitis B-Virus kodiert, dadurch gekennzeichnet, dass es ein Oligo- oder Polypeptid gemäß einem der Gegenstände (8) bis (9) enthält.
(13) Monoklonaler oder polyklonaler Antikörper, der an HBs-Antigen, enthaltend ein Oligo- oder Polypeptid gemäß einem der Gegenstände (8) bis (12) bindet, der an HBs-Antigen eines Hepatitis B-Wildtypvirus aber nicht oder wenigstens signifikant schwächer bindet.
(14) Testkit zum Nachweis oder zur Bestimmung mittels einer Hybridisierungsreaktion einer Nukleinsäure, die für eine Variante oder Mutante des Hepatitis B-Virus spezifisch ist, unter Verwendung mindestens eines Oligo- oder Polynukleotid gemäß einem oder mehreren der Gegenstände (1) bis (5).
(15) Testkit zum immunchemischen Nachweis oder zur immunchemischen Bestimmung eines Antigens, das für eine Variante oder Mutante des Hepatitis B-Virus spezifisch ist, unter Verwendung mindestens eines monoklonalen oder polyklonalen Antikörpers gemäß (13).
(16) Testkit zum immunchemischen Nachweis oder zur immunchemischen Bestimmung eines gegen eine Variante oder Mutante des Hepatitis B-Virus gerichteten Antikörpers unter Verwendung mindestens eines Oligo- oder Polypeptids gemäß einem der Gegenstände (8) bis (12).

Zusätzlich beinhaltet die vorliegende Erfindung eine isolierte Nukleotid-Sequenz, die die vorliegende erfindungsgemäße Variante der a-Determinante des Hepatitis B surface Antigens (HBcAg) in den Aminosäuren-Positionen zwischen aa 101 und 180 kodiert

Des weiteren beeinhaltet die vorliegende Erfindung einen Vektor, umfassend eine oder mehrere der genannten Nukleotid-Sequenzen wie auch eine Wirtszelle, die diesen Vektor enthält und eine Methode zur Darstellung eines entsprechenden Polypeptids aus der a-Determinante, umfassend die Inkubation der oben genannten Wirtszelle über Zeiten und unter Bedingungen, die für die Expression des Polypeptides erforderlich sind.

Auch Gegenstand der Erfindung sind Antikörper, die mit der in SEQ ID NO:12 bis 30 beschriebenen a-Determinante reagieren, wobei die Bindung bevorzugt in dem Aminosäurebereich aa 101 bis 150 erfolgt. Die Antikörper können polyklonalen oder monoklonalen tierischen oder menschlichen Ursprungs sein.

Auch eine immunogene Mischung zur Erzeugung polyklonaler oder monoklonaler Antikörper ist Gegenstand der vorliegenden Erfindung umfassend das beschriebene isolierte HBV oder ein bzw. mehrere der beschriebenen Poypeptide.

Die Erfindung beinhaltet auch eine Polynukleotid-Sonde, enthaltend eine HBV Genom-Sequenz, welche durch Substitution von Aminosäuren zu einer modifizierten a-Determinanten führt, die mit der beschriebenen aa-Sequenz der neuen HBV-Variante identisch ist.

Auch Kits zum Nachweis von Polynukleotiden der HBV-Variante mit Hilfe der genannten Sonde wie auch Kits zum Nachweis von HBsAg der Variante bzw. einzelnen Epitopen davon und Antikörper, die für die Variante oder Epitope davon spezifisch sind, sind ebenso Gegenstand der Erfindung, wie die Methoden des Nachweises von Polynukleotiden, Antigen und Antikörper, umfassend eine Inkubation zur Bildung entsprechender Komplexe und Nachweis dieser komplexe durch geeignete dem Fachmann bekannte Verfahren.

Die Ausführungsformen dieser Kits und Nachweismethoden können zum spezifischen und alleinigen Nachweis von Nukleotiden und Antigen der HBV-Variante bzw. dagegen gerichteter Antikörper ausgelegt sein oder supplementär, d.h. den zusätzlichen Nachweis der erfindungsgemäßen Varianten-Analyten zu derzeitig bekannten HBV-Nukleotiden, - Antigenen bzw. - Antikörpern gestatten.

Analog kann eine immunogene Mischung von erfindungsgemäßen Polypeptidsequenzen auch in Verbindung mit bekannten Antigenen z.B. für die Verbesserung der Wirksamkeit einer Vakzine angewendet werden.

Die vorliegende Erfindung beschreibt eine neue Variante des Hepatitis B Virus (HBV), die eine völlig neue a-Determinante als Resultat von Aminosäure-Austauschen in den nachfolgenden aa-Positionen der S-HBsAg Sequenz aufweist. Für die Beschreibung der Aminosäuren wird der 1-Buchstaben-Code verwendet:

| aa von HDB 11 | aa-Position | aa von ayw2/Genotyp D |
|---|---|---|
| I | 103 | M |
| A | 114 | S |
| I | 115 | T |
| N | 116 | T |
| N | 117 | S |
| R | 118 | T |
| Q | 120 | P |
| T | 127 | P |
| H | 129 | Q |
| Y | 136 | S. |

Außerdem liegt in Position aa 96 von HDB 11 Alanin (A) statt Valin (V) vor:

| | | |
|---|---|---|
| A | 96 | V |

Diese aa-Substitutionen lassen sich auf entsprechende Nukleotid-Substitutionen der entsprechenden Codons zurückführen.

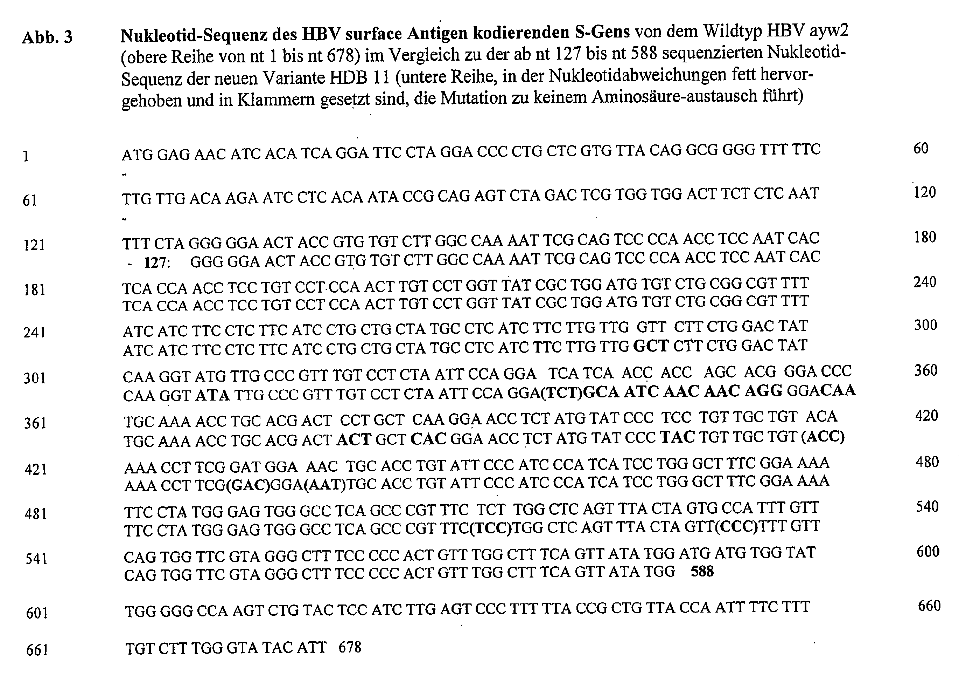

Die vorliegende Erfindung betrifft eine isolierte Nukleotid-Sequenz, die für die a-Determinante des Virus kodiert (Abb. 3 sowie SEQ ID No.: 1).

Für die Beschreibung der vorliegenden Erfindung wird unter einem Nukleotid-Fragment eine konsekutive Folge von bevorzugt 21-60 Nukleotide aus der Nukleotidsequenz der neuen HBV-Variante verstanden, wobei auch Mischungen derartiger Nukleotid-Fragmente umfasst sind.

Unter einem Polypeptid-Fragment wird eine Folge von bevorzugt 7-20 Aminosäuren aus der a-Determinante der neuen HBV-Variante verstanden, wobei auch Mischungen derartiger Polypeptid-Fragmente unter diese Erfindung fallen.

Unter die vorliegende Erfindung fällt auch eine isolierte Nukleotid-Sequenz, die hybridisierbar ist und zu Nukleotid-Sequenzen führt, die den Nukleotidsequenze des HBsAg der neuen HBV Variante oder Teilen der a-Determinante der neuen HBV Variante entsprechen, komplementär dazu sind oder als Subtyp oder Mutation auf HDB 11 zurückzuführen sind.

Dem Fachmann ist bekannt, dass eine Nukleotidsequenz nach deren Isolierung nach Methoden gemäß Stand der Technik in prokaryontische (z.B. E. coli), eukaryontische Wirtszellen (z.B. Chinese Hamster Ovary Cell) oder Hefe (z.B. S. Cerevisiae) mit Hilfe eines Vektors oder Konstruktes eingebracht werden können (mit dem Fachmann bekannten Methoden wie z.B. Transfektion, Transformation oder Elektroporation: Molecular Cloning: A Laboratory Manual, 2nd ed., Vol. 1-3, ed Sambrook et al., Cold Spring Harbor Laboratory Press (1989), wobei transiente oder permanente Kulturen angewendet werden können. Demzufolge umfasst die vorliegende Erfindung isolierte Nukleotidsequenzen der a-Determinante der neuen HBV Variante, Polypeptide, die durch diese Nukleotide kodiert werden, Vektoren, die Nukleotidsequenzen der a-Determinante der neuen HBV Variante enthalten wie auch die Wirtszelle, in die ein Vektor gebracht wird. Neben der Darstellung von Polypeptiden mit Hilfe eines Expressions-Systems (rekombinant oder gentechnologisch) ist dem Fachmann bekannt, dass analoge Polypeptid-Strukturen auch vollsynthetisch oder direkt durch Reinigung aus der Virusvariante hergestellt werden können.

Es ist möglich, die Polypeptide oder Proteine der neuen HBV Variante zur Erzeugung von monoklonalen und/oder polyklonalen Antikörpern zu verwenden, die immunologisch an Bindestellen (Epitope) der a-Determinante der neuen HBV Variante binden. Die Methoden zur Darstellung von Antikörpern sind dem Fachmann bekannt (z.B. Koehler et al., Nature 256-494 (1975), Mimms et al., Vi. 176: 604-619 (1990).

Ferner ist es möglich, die a-Determinante der erfindungsgemäßen HDB 11-Variante in Form der gesamten Polypeptidsequenz oder Teilen davon für die Bestimmung von gegen die HBV Variante gerichteten Antikörpern (Anti-HBs-Antikörpern) zu verwenden (siehe oben).

Dem Fachmann ist eine Vielzahl von Bestimmungsmethoden geläufig, bei denen mit Polypeptiden aus der a-Determinante der HBV Variante und Antikörpern tierischen oder menschlichen Ursprungs Immunkomplexe gebildet oder deren Bildung gehemmt werden.

Schließlich ist es möglich, monoklonale oder polyklonale Antikörper (oder Mischungen bzw. Fragmente davon oder Mischungen von Fragmenten), die mit Epitopen der neuen HBV Variante reagieren, dazu zu verwenden, die a-Determinante der erfindungsgemäßen HBV Variante in Form der gesamten Polypeptidsequenz oder Teilen davon in Untersuchungsproben zu bestimmen: HBsAg der HDB 11-Variante.

Dem Fachmann ist eine Vielzahl von Bestimmungsmethoden geläufig, bei denen mit einem oder mehreren monoklonalen Antikörper(n) oder polyklonalen Antikörpern (oder Mischungen davon bzw. Fragmenten oder Mischungen von Fragmenten), die spezifisch für die a-Determinante der HBV Variante sind, Immunkomplexe gebildet oder deren Bildung gehemmt werden.

Ebenso können auf Basis der gefundenen Nukleotidsequenzen der neuen HBV-Variante entsprechende Primer entwickelt werden.

Schließlich sind auch diagnostische Reagenzien als Kits Gegenstand der Erfindung, die basierend auf den oben beschriebenen Verfahren einen Nachweis von HBV Variantenspezifischem Antigen (HBsAg) oder dagegen gerichtete Antikörper (Anti-HBs), entweder als singuläre Bestimmungen oder miteinander oder mit anderen bekannten HBV-Antigen bzw. spezifisch damit reagierenden Antikörpern bzw. auch mit ganz anderen Analyten kombinierbar.

Die vorliegende Erfindung ist darüber hinaus in den Patentansprüchen beschrieben.

### Beschreibung der Abbildungen:

**Abb. 1** stellt eine Übersicht der Aminosäure- Sequenzen der a-Determinante von 6 beschriebenen Genotypen des HBV im Vergleich zu HDB 11 dar.
In **Abb. 2** sind die Nukleotid- und Aminosäure-Sequenzen der a-Determinante sowie unmittelbar benachbarter Regionen des Genotyps D, Subtyp ayw2 von HBV dargestellt.
**Abb. 3** zeigt die Nukleotid-Sequenz der a-Determinante des HBV surface Antigens für Subtyp ayw2 des Genotyps D von HBV im Vergleich zur Nukleotid-Sequenz von HDB 11.

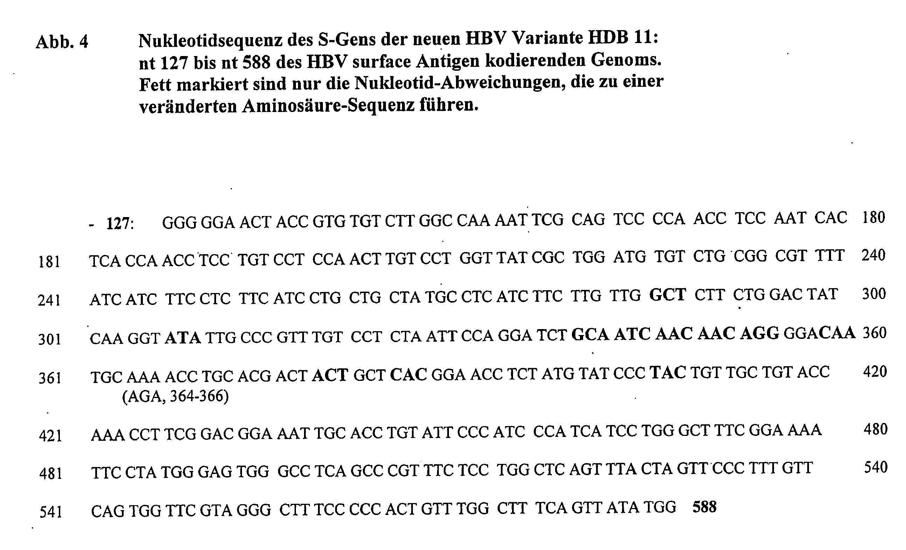

**Abb. 4** fasst die Translations-relevanten Abweichungen der Nukleotid-Sequenz von HDB 11 zusammen.

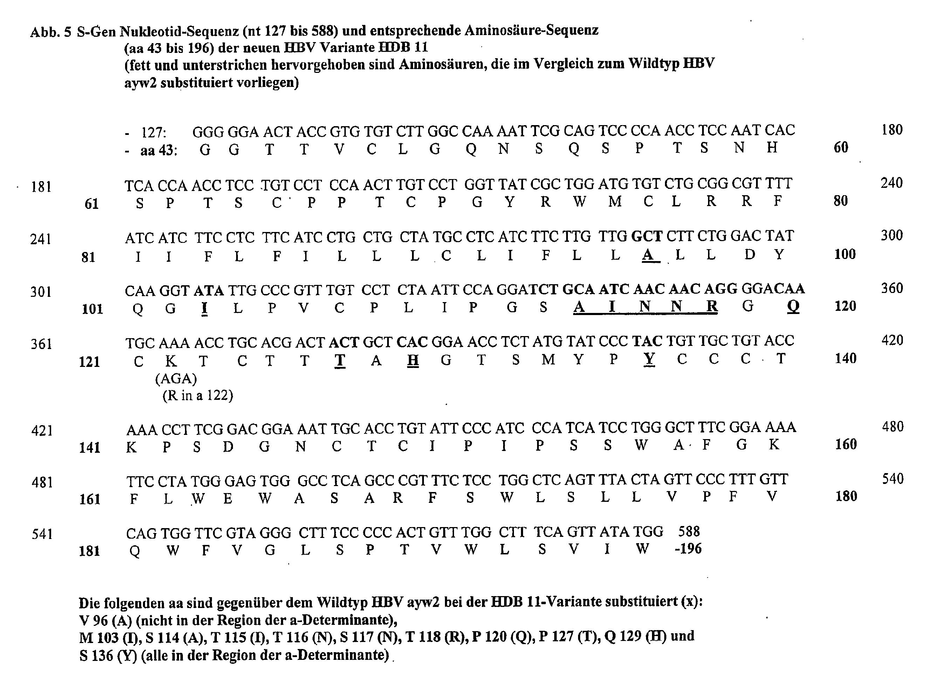

In **Abb. 5** wird die Nukleotid-Sequenz von HDB 11 in der Region der a-Determinante sowie die entsprechende Aminosäure-Sequenz dargestellt. Die a-Determinante befindet sich zwischen Aminosäure No. 101 und 180 des kleinen HBsAg (Small, S).

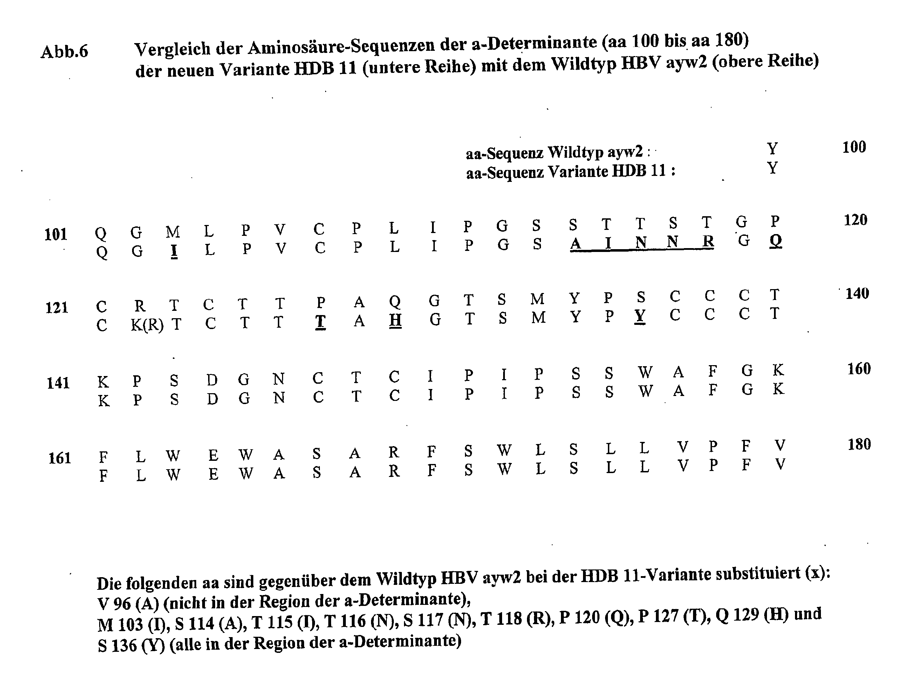

**Abb. 6** zeigt die entsprechende Polypeptid-Sequenz der a-Determinante von HDB 11, die von der in Abb. 5 beschriebenen Nukleotid-Sequenz kodiert wird.

Die nachfolgenden Beispiele erläutern die vorliegende Erfindung näher, ohne dass die Erfindung auf die beschriebenen Beispiele beschränkt ist.

### Beispiel 1: HBsAg-Bestimmung mittels Enzymimmunoassay, EIA

Zur Bestimmung des surface Antigens von HBV, HBsAg im Blut des ägyptischen Patienten wurde der Enzymimmunoassay Enzygnost ® HBsAg 5.0 der Fa. Dade Behring GmbH, Marburg Deutschland angewendet.

Es handelt sich um einen in Europa zugelassenen und leistungsfähigen Test, der den Angaben der Packungsbeilage gemäß abgearbeitet wurde.

Das zu Grunde liegende Testprinzip ist ein sogenannter Sandwich Test im Mikrotiterplatten-Format:

100 µl der zu untersuchenden Probe werden in einem Einschritt-Verfahren mit 25 µl Konjugat 1 (monoklonale HBsAg spezifische Antikörper von der Maus, die kovalent mit Biotin markiert sind) und immobilisierten HBsAg-spezifischen polyklonalen Antikörpern vom Schaf in Kontakt gebracht. Nach 60-minütiger Inkubation bei 37°C und Entfernen überschüssiger Komponenten durch 4-maliges Waschen der Platten-Kavitäten werden 100 µl Konjugat 2 zugegeben, das aus Streptavidin besteht, an das das Sonden-Enzym Peroxidase kovalent gebunden ist.
Nach 30-minütiger Inkubation bei 37°C und Entfernen überschüssiger Komponenten durch 4-maliges Waschen der Platten-Kavitäten werden 75 µl Chromogen-Puffer/Substrat-Lösung zugegeben, gefolgt von einer 30-minütigen Inkubation bei Raumtemperatur. Die Entwicklung des blau gefärbten Tetramethylbenzidin-Farbstoffes wird durch Zugabe von 75 µl Stoplösung (Schwefelsäure) unterbrochen und der Farbstoff bei 450 nm photometrisch gemessen.

Die Intensität der Farbentwicklung, gemessen an der optischen Dichte (O.D.) ist dem Gehalt der Untersuchungsprobe an HBsAg direkt proportional, wobei ein O.D. Wert von kleiner als dem Grenzwert als HBsAg-negativ bewertet wird. Der Grenzwert ist definiert als der O.D.-Mittelwert der parallel getesteten Kontrolle, negativ (im Testkit enthalten), zu dem ein konstanter Betrag von 0,05 O.D. addiert wird.

Die Nachweisgrenzen des zur Untersuchung herangezogenen Lots (# 32874) wurden mit den international akzeptierten Standardpräparationen des Paul-Ehrlich-Institutes, Langen Deutschland zu 0,012 ng ad-Subtyp/ ml bzw. 0,015 ng ay-Subtyp / ml in parallelen Versuchsansätzen aus Testungen von Verdünnungen der Standardpräparationen in HBsAgnegativem Serum durch graphische Interpolation ermittelt.

Die Untersuchung der Probe # 118234 (Entnahme vom 02. Okt. 2002, aus der auch die DNA-Isolierung vorgenommen wurde) brachte in 2 unabhängigen Versuchen an zwei verschiedenen Tagen Ergebnisse von 0,04 und 0,05 O.D., die den Kriterien des Testes entsprechend als HBsAg-negativ zu interpretieren sind. Die mitgeführte Kontrolle, positiv (im Testkit enthalten) war dagegen ebenso positiv (Validierungskriterien erfüllt) wie die erwähnten ad- und ay-Standardpräparationen.

### Beispiel 2: Isolierung der HDB 11- DNA aus Probe # 118234

Aus einem 200 µl-Aliquot der ägyptischen Probe wurde die DNA isoliert, indem der QIA amp® DNA Blood Mini Kit der Fa. Qiagen, Hilden Deutschland) angewendet wurde. Dabei wurden alle Verfahrensschritte wie in der Packungsbeilage beschrieben befolgt und die Elution in einem Volumen von 50 µl vorgenommen.

### Beispiel 3: Polymerase Ketten Reaktion, PCR

### 3.1 HBV Primer

Die vier nachstehenden HBV Primer wurden verwendet:
Primer 1 mit der 5'> 3'- Sequenz: GGGTCACCATATTCTTGGGAAC (SEQ ID NO:31)
Primer 2 mit der 5'> 3'- Sequenz: TATACCCAAAGACAAAAGAAAATTGG (SEQ ID NO:32)
Primer 3 mit der 5'> 3'- Sequenz: GACTCGTGGTGGACTTCTCTC (SEQ ID NO:33)
Primer 4 mit der 5'> 3'- Sequenz: TACAGACTTGGCCCCCAATACC (SEQ ID NO:34)

### 3.2 PCR-Amplifikation

Es wurde eine sogenannte nested PCR amplification des surface Antigens durchgeführt, wobei der Perkin Elmer Ampli Taq ® DNA Polymerase Kit sowie der Thermocycler Gene Amp ® PCR system 9700 der Fa. Perkin Elmer Applied Biosystems, USA verwendet wurde.

Die Nukleotide wurden von der Fa. Amersham Biosciences, UK bezogen.

Für den ersten Amplifikations-Zyklus wurden 5 µl der isolierten DNA unter Verwendung der oben genannten Primer 1 und 2 sowie folgenden Bedingungen amplifiziert:

### PCR 1 rxn

| | | |
|---|---|---|
| Primer 1 (10 µM) | 1 µl | |
| Primer 2 (10 µM) | 1 µl | |
| 10fach konz. Puffer (incl. 15 µM Mg2Cl) | 5 µl | |
| dNTP Mischung (10 µM) | 1 µl | |
| dest. Wasser | 36,75 µl | |
| Ampli Taq (5 U/ µl) | 0.25 µl | |
| Pro Röhrchen | 45 µl | Gesamtvolumen |
| plus | 5 µl | isolierte DNA |
| | 50 µl | Reaktionsvolumen |

Der 50 µl-Ansatz wurde unter Verwendung des beschriebenen Thermocyclers unter folgenden Bedingungen amplifiziert:
94° C, 1 min. / 94°C, 28 sek. - 50° C, 28 sek. - 72 ° C, 38 sek. (35 cycles) / 72 ° C, 5 min. / 8 °C soak.

In der zweiten Amplifizierungsrunde wurde 5 µl des ersten PCR . Produktes weiter amplifiziert unter Verwendung der HBV Primer 3 und 4 und folgenden Bedingungen:

### PCR 2 rxn

| | | |
|---|---|---|
| Primer 3 (10 µM) | 1 µl | |
| Primer 4 (10 µM) | 1 µl | |
| 10 -fach konz. Puffer | 5 µl | |
| dNTP Mischung (10 µM) | 1 µl | |
| dest. Wasser | 36,75 µl | |
| Ampli Taq (5 U/ µl) | 0,25 µl | |
| Pro Röhrchen 45 µl | Gesamtvolumen | |
| plus | 5 µl | PCR Produkt v.rxn |
| | 50 µl | Reaktionsvolumen |

Dieser PCR 2 -Ansatz wurde unter Verwendung des oben beschriebenen Thermocyclers amplifiziert , wobei folgende Bedingungen angewendet wurden:
94° C, 1 min. / 94°C, 28 sek. - 55° C, 28 sek. - 72 ° C, 38 sek. (35 cycles) / 72 ° C, 5 min. / 8 °C soak.

Abschließend wurde das PCR 2 Produkt elektrophoretisch aufgetrennt (1,5 % Agarose) unter Mitführen geeigneter Molekulargewichtsmarker. Die Bande mit ca. 520 Basenpaaren wurde ausgeschnitten und mit Hilfe.des QIA quick Gel Extraction Kit der Fa. Qiagen, Hilden Deutschland isoliert .

### Beispiel 4: Sequenzierung von HDB 11

Das gereinigte PCR Produkt wurde von der Fa. Medigenomix, Martinsried, Deutschland mit Hilfe des ABI 3700 Kapillar Systems sequenziert in Verbindung mit der ABI BigDye Terminator Chemistry Version 1.1. und der ABI Sequencing Analysis Software Version 3.6. unter Verwendung der in Beispiel 3 beschriebenen Primer 3 und 4.

### Sequenzierungs-Ergebnis

Es konnte gezeigt werden, dass das HBsAg der Probe innerhalb des sequenzierten Bereiches die beste Übereinstimmung der Nukleotid- und Aminosäure-Sequenz mit dem Genotyp D, Subtyp ayw2 zeigt, aber in der Region der a-Determinante insgesamt 10 Aminosäure-Substitutionen aufweist (siehe auch Abb. 2 und 5):

| **HDB 11:** | | **D, ayw2:** |
|---|---|---|
| 1.) | Ile (I) | gegen 103 Met (M) |
| 2.) | Ala (A) | gegen 114 Ser (S) |
| 3.) | Ile (I) | gegen 115 Thr (T) |
| 4.) | Asn (N) | gegen 116 Thr (T) |
| 5.) | Asn (N) | gegen 117 Ser (S) |
| 6.) | Arg (R) | gegen 118 Thr (T) |
| 7.) | Gln (Q) | gegen 120 Pro (P) |
| 8.) | Thr (T) | gegen 127 Pro (P) |
| 9.) | His (H) | gegen 129 Gln (Q) |
| 10.) | Tyr (Y) | gegen 136 Ser (S) |

Zusätzlich liegt eine Aminosäure-Substitution in der Position # 96 vor:

| | | |
|---|---|---|
| 11.) | Ala (A) gegen | 96Val (V) |

Diese Ergebnisse wurden in mehreren unabhängigen Versuchen aus verschiedenen Primär-Röhrchen der Blutentnahme vom 02. Okt. 2002 mit den gleichen Sequenzierungs-Ergebnissen reproduziert.

Als einzige Ausnahme wurde in der ersten Analyse die Position aa# 122 als Arg R gelesen, während die zweite Analyse eher für Lys (K) sprach.
Das Nukleotid-Profil läßt beide Interpretationen zu, was allerdings auch Hinweis für das Vorliegen der Koexistenz zweier Hepatitis B Viren mit unterschiedlicher Subtypisierung (ad bzw. ay) sprechen könnte, aber an der Aminosäure-Sequenz bzw. dem Rückschluß auf das Vorhandensein einer neuen Variante mit oben genannten Aminosäure-Substitutionen nichts ändert.
Dies wird unter anderem aus dem negativen EIA Ergebnis geschlossen, da nennens-werte Mengen zirkulierenden HBsAg bekannter Struktur im Hinblick auf die sehr guten Nachweisgrenzen der angewendeten EIA Bestimmungsmethode in jedem Fall Anlaß zu einem positiven EIA-Ergebnis hätte geben müssen.

Die Aminosäure, die Position 122 entspricht, kann daher in den Sequenzen SEQ ID NO:12, SEQ ID NO:14, SEQ ID NO:15, SEQ ID NO:16, SEQ ID NO:25, SEQ ID NO:27, SEQ ID NO:28, SEQ ID NO:29 und SEQ ID NO:30 entweder K oder R sein.

## Patentansprüche

1. Oligo- oder Polypeptid, **dadurch gekennzeichnet, dass** es eine Aminosäuresequenz umfasst, die ausgewählt ist aus der Gruppe bestehend aus SEQ ID NO:12, SEQ ID NO:13, SEQ ID NO:14, SEQ ID NO:15, SEQ ID NO:16, SEQ ID NO:26, SEQ ID NO:27, SEQ ID NO:28 und SEQ ID NO:29.

2. Oligo- oder Polynukleotid umfassend eine Nukleotidsequenz, die für ein Oligo- oder Polypeptid nach Anspruch 1 kodiert oder ein dazu komplementäres Oligo- oder Polynukleotid.

3. Oligo- oder Polynukleotid nach Anspruch 2, **dadurch gekennzeichnet, dass** es eine Nukleotidsequenz umfasst, die ausgewählt ist aus der Gruppe bestehend aus SEQ ID NO: 1, SEQ ID NO:2, SEQ ID NO:3, SEQ ID NO:4, SEQ ID NO:5, SEQ ID NO:7, SEQ ID NO:8, SEQ ID NO:9 und SEQ ID NO:10.

4. Oligo- oder Polynukleotid nach Anspruch 2, **dadurch gekennzeichnet, dass** es eine Länge von 10 bis 30 Nukleotiden hat.

5. Vektor oder Plasmid enthaltend ein Oligo- oder Polynukleotid nach einem der Ansprüche 2 oder 3.

6. Zelle, die mit einem Vektor oder Plasmid nach Anspruch 5 transformiert oder transfiziert wurde.

7. Zelle, die ein Oligo- oder Polynukleotid nach einem der Ansprüche 2 oder 3 oder einen Vektor oder ein Plasmid nach Anspruch 5 enthält.

8. Verfahren zur Herstellung eines Oligo- oder Polypeptids nach Anspruch 1, das umfasst, dass man eine Zelle nach Anspruch 6 oder 7 unter geeigneten Bedingungen kultiviert, so dass das Oligo- oder Polypeptid exprimiert wird.

9. Verfahren nach Anspruch 8, **dadurch gekennzeichnet, dass** das Oligo- oder Polypeptid aus den Zellen gewonnen wird und von anderen Oligo- oder Polypeptiden abgetrennt wird.

10. Antikörper, der an die in SEQ ID NO:12 beschriebenen a-Determinante im Aminosäurebereich zwischen Position 101 bis 150 bindet.

11. Antikörper nach Anspruch 10, **dadurch gekennzeichnet, dass** er an HBs-Antigen enthaltend ein Oligo- oder Polypeptid nach Anspruch 1 bindet, aber nicht oder signifikant schwächer an HBs-Antigen eines Hepatitis B-Virus Genotyp D, Subtyp ayw2.

12. Testkit zum Nachweis von Hepatitis B-Viren, enthaltend
(i) ein Oligo- oder Polypeptid nach Anspruch 1;
(ii) ein Oligo- oder Polynukleotid nach einem der Ansprüche 2 oder 3; und/oder
(iii) einen Antikörper nach einem der Ansprüche 10 oder 11.

13. lmmunogenes Peptid, enthaltend ein oder mehrere Oligo- oder Polypeptide nach Anspruch 1 alleine oder in Verbindung mit bekannten HBV-Immunogenen.

14. Mischung immunogener Peptide, enthaltend ein oder mehrere Oligo- oder Polypeptide nach Anspruch 1 alleine oder in Verbindung mit bekannten HBV-Immunogenen.

15. Ein in vitro Verfahren zum Nachweis eines Hepatitis B-Antigens, **dadurch gekennzeichnet, dass**
(a) eine Probe mit einem Antikörper nach Anspruch 10 oder 11 unter Bedingungen inkubiert wird, die die Bildung eines Antigen-Antikörper-Komplexes gestatten; und
(b) ein Antigen-Antikörper-Komplex, der den Antikörper enthält, nachgewiesen wird.

16. Ein in vitro Verfahren zum Nachweis von Antikörpern, die gegen ein Hepatitis B-Antigen gerichtet sind, **dadurch gekennzeichnet, dass**
(a) eine Probe mit einem Oligo- oder Polypeptid nach Anspruch 1 unter Bedingungen inkubiert wird, die die Bildung eines Antigen-Antikörper-Komplexes gestatten; und
(b) der Antikörper-Antigen-Komplex, der das Oligo- oder Polypeptid enthält, nachgewiesen wird.

17. Ein in vitro Verfahren zum Nachweis einer Hepatitis B-Nukleinsäure, **dadurch gekennzeichnet, dass**
(a) eine Probe mit einem Oligo- oder Polynukleotid nach einem der Ansprüche 2 oder 3 unter Bedingungen inkubiert wird, die die selektive Hybridisierung des Oligo- oder Polynukleotids mit einer Hepatitis B-Nukleinsäure in der Probe gestatten; und
(b) bestimmt wird, ob Polynukleotidduplexe gebildet wurden, die das Oligo- oder Polynukleotid umfassen.

18. Ein in vitro Verfahren zum Nachweis einer Hepatitis B-Nukleinsäure, **dadurch gekennzeichnet, dass**
(a) eine Probe mit wenigstens einem Oligo- oder Polynukleotid nach einem der Ansprüche 2 oder 3 unter Bedingungen inkubiert wird, die die selektive Hybridisierung des Oligo- oder Polynukleotids mit einer Hepatitis B-Nukleinsäure in der Probe gestatten;
(b) eine Polymerasekettenreaktion durchgeführt wird; und
(c) bestimmt wird, ob eine Nukleinsäure amplifiziert wurde.

19. Verwendung eines Oligo- oder Polynukleotids nach einem der Ansprüche 2 oder 3 als Primer.

20. Verwendung eines Oligo- oder Polynukleotids nach einem der Ansprüche 2 oder 3 als Sonde.

## Claims

1. Oligopeptide or polypeptide, **characterized in that** it comprises an amino acid sequence which is selected from the group consisting of SEQ ID NO: 12, SEQ ID NO: 13, SEQ ID NO: 14, SEQ ID NO: 15, SEQ ID NO: 16, SEQ ID NO: 26, SEQ ID NO: 27, SEQ ID NO: 28 and SEQ ID NO: 29.

2. Oligonucleotide or polynucleotide comprising a nucleotide sequence which encodes an oligopeptide or polypeptide according to Claim 1 or an oligonucleotide or polynucleotide which is complementary thereto.

3. Oligonucleotide or polynucleotide according to Claim 2, **characterized in that** it comprises a nucleotide sequence which is selected from the group consisting of SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9 and SEQ ID NO: 10.

4. Oligonucleotide or polynucleotide according to Claim 2, **characterized in that** it has a length of from 10 to 30 nucleotides.

5. Vector or plasmid which contains an oligonucleotide or polynucleotide according to either of claims 2 and 3.

6. Cell which has been transformed or transfected with a vector or plasmid according to Claim 5.

7. Cell which contains an oligonucleotide or polynucleotide according to either of Claims 2 and 3 or a vector or plasmid according to Claim 5.

8. Method for preparing an oligopeptide or polypeptide according to Claim 1 which comprises culturing a cell according to Claim 6 or 7 under suitable conditions such that the oligopeptide or polypeptide is expressed.

9. Method according to Claim 8, **characterized in that** the oligopeptide or polypeptide is isolated from the cells and separated off from other oligopeptides or polypeptides.

10. Antibody which binds to the a determinant described in SEQ ID NO: 12 in the amino acid region between position 101 to 150.

11. Antibody according to Claim 10, **characterized in that** it binds to an HBs antigen which contains an oligopeptide or polypeptide according to Claim 1 but not, or significantly more weakly, to HBs antigen belonging to a genotype D, subtype ayw2, hepatitis B virus.

12. Test kit for detecting hepatitis B viruses, comprising
(i) an oligopeptide or polypeptide according to Claim 1;
(ii) an oligonucleotide or polynucleotide according to either of Claims 2 and 3; and/or
(iii) an antibody according to either of claims 10 and 11.

13. Immunogenic peptide containing one or more oligopeptides or polypeptides according to Claim 1 on its/their own or in combination with known HBV immunogens.

14. Mixture of immunogenic peptides containing one or more oligopeptides or polypeptides according to Claim 1 on its/their own or in combination with known HBV immunogens.

15. In vitro method for detecting a hepatitis B antigen, **characterized in that**
(a) a sample is incubated with an antibody according to Claim 10 or 11 under conditions which allow the formation of an antigen-antibody complex; and
(b) an antigen-antibody complex which contains the antibody is detected.

16. In vitro method for detecting antibodies which are directed against a hepatitis B antigen, **characterized in that**
(a) a sample is incubated with an oligopeptide or polypeptide according to Claim 1 under conditions which allow the formation of an antigen-antibody complex; and
(b) the antibody-antigen complex which contains the oligopeptide or polypeptide is detected.

17. In vitro method for detecting a hepatitis B nucleic acid, **characterized in that**
(a) a sample is incubated with an oligonucleotide or polynucleotide according to either of Claims 2 and 3 under conditions which allow the selective hybridization of the oligonucleotide or polynucleotide with a hepatitis B nucleic acid in the sample; and
(b) it is determined whether polynucleotide duplexes which comprise the oligonucleotide or polynucleotide have been formed.

18. In vitro method for detecting a hepatitis B nucleic acid, **characterized in that**
(a) a sample is incubated with at least one oligonucleotide or polynucleotide according to either of Claims 2 and 3 under conditions which allow the selective hybridization of the oligonucleotide or polynucleotide with a hepatitis B nucleic acid in the sample;
(b) a polymerase chain reaction is carried out; and
(c) it is determined whether a nucleic acid has been amplified.

19. Use of an oligonucleotide or polynucleotide according to either of Claims 2 and 3 as a primer.

20. Use of an oligonucleotide or polynucleotide according to either of Claims 2 and 3 as a probe.

## Revendications

1. Oligopeptide ou polypeptide, **caractérisé en ce qu'**il comprend une séquence d'acide aminé, qui est choisie dans le groupe constitué de l'identification de séquence N° 12, de l'identification de séquence N° 13, de l'identification de séquence N° 14, de l'identification de séquence N° 15, de l'identification de séquence N° 16, de l'identification de séquence N° 26, de l'identification de séquence N° 27, de l'identification de séquence N° 28 et de l'identification de séquence N° 29.

2. Oligonucléotide ou polynucléotide, comprenant une séquence de nucléotide, qui code pour un oligopeptide ou un polypeptide suivant la revendication 1 ou pour un oligonucléotide ou un polynucléotide complémentaire de celui-ci .

3. Oligonucléotide ou polynucléotide suivant la revendication 2, **caractérisé en ce qu'**il comprend une séquence de nucléotide, qui est choisie dans le groupe constitué de l'identification de séquence N° 1, de l'identification de séquence N° 2, de l'identification de séquence N° 3, de l'identification de séquence N° 4, de l'identification de séquence N° 5, de l'identification de séquence N° 7, de l'identification de séquence N° 8, de l'identification de séquence N° 9 et de l'identification de séquence N° 10.

4. Oligonucléotide ou polynucléotide suivant la revendication 2, **caractérisé en ce qu'**il a une longueur de 10 à 30 nucléotides.

5. Vecteur ou plasmide contenant un oligonucléotide ou un polynucléotide suivant l'une des revendications 2 ou 3.

6. Cellule, qui est transformée ou transfectée par un vecteur ou par un plasmide suivant la revendication 5.

7. Cellule, qui contient un oligonucléotide ou un polynucléotide suivant l'une des revendications 2 ou 3 ou un vecteur ou un plasmide suivant la revendication 5.

8. Procédé de production d'un oligopeptide ou d'un polypeptide suivant la revendication 1, qui comprend le fait de cultiver une cellule suivant la revendication 6 ou 7 dans des conditions appropriées, de manière à exprimer l'oligopeptide ou le polypeptide.

9. Procédé suivant la revendication 8, **caractérisé en ce qu'**on obtient l'oligopeptide ou le polypeptide à partir des cellules et on le sépare d'autres oligopeptides ou polypeptides.

10. Anticorps, qui se fixe aux déterminants a, décrits dans l'identification de séquence N° 12, de la région d'acide aminé entre les positions 101 à 150.

11. Anticorps suivant la revendication 10, **caractérisé en ce qu'**il se fixe à un antigène d'HBs contenant un oligopeptide ou un polypeptide suivant la revendication 1, mais pas ou d'une manière significativement plus faible à un antigène HBs d'un virus de l'hépatite B, génotype D, sous-type ayw2.

12. Trousse de test pour la détection de virus de l'hépatite B, contenant :
(I) un oligopeptide ou un polypeptide suivant la revendication 1 ;
(II) un oligonucléotide ou un polynucléotide suivant l'une des revendications 2 ou 3 et/ou
(III) un anticorps suivant l'une des revendications 10 ou 11.

13. Peptide immunogène, contenant un ou plusieurs oligopeptides ou polypeptides suivant la revendication 1, seul ou en liaison avec des immunogènes HBV connus.

14. Mélange de peptides immunogènes, contenant un ou plusieurs oligopeptides ou polypeptides suivant la revendication 1, seul ou en liaison avec des immunogène HBV connus.

15. Procédé in vitro de détection d'un antigène de l'hépatite B, **caractérisé en ce que**
(a) on fait incuber un échantillon avec un anticorps suivant la revendication 10 ou 11 dans des conditions qui autorisent la formation d'un complexe antigène-anticorps et
(b) on détecte un complexe antigène-anticorps qui contient l'anticorps.

16. Procédé in vitro de détection d'anticorps qui sont dirigés contre l'antigène de l'hépatite B, **caractérisé en ce que**
(a) on fait incuber un échantillon avec un oligopeptide ou un polypeptide suivant la revendication 1 dans des conditions qui autorisent la formation d'un complexe antigène-anticorps et
(b) on détecte un complexe antigène-anticorps qui contient l'oligopeptide ou le polypeptide.

17. Procédé in vitro de détection d'un acide nucléique de l'hépatite B, **caractérisé en ce que**
(a) on fait incuber un échantillon avec un oligonucléotide ou un polynucléotide suivant l'une des revendications 2 ou 3 dans des conditions qui autorisent l'hybridation sélective de l'oligonucléotide ou du polynucléotide avec un acide nucléique de l'hépatite B de l'échantillon et
(b) on détermine si un duplex de polynucléotide a été formé qui comprend l'oligonucléotide ou le polynucléotide.

18. Procédé in vitro de détection d'un acide nucléique de l'hépatite B, **caractérisé en ce que**
(a) on fait incuber un échantillon avec au moins un oligonucléotide ou un polynucléotide suivant l'une des revendications 2 ou 3 dans des conditions qui autorisent l'hybridation sélective de l'oligonucléotide ou du polynucléotide avec un acide nucléique de l'hépatite B de l'échantillon ;
(b) on effectue une réaction par amplification génique en chaîne et
(c) on détermine si un acide nucléique a été amplifié.

19. Utilisation d'un oligonucléotide ou d'un polynucléotide suivant l'une des revendications 2 ou 3 comme amorce.

20. Utilisation d'un oligonucléotide ou d'un polynucléotide suivant l'une des revendications 2 ou 3 comme sonde.
